(19) Europäisches Patentamt
European Patent Office
Office européen des brevets

(11) **EP 1 915 984 A1**

(12) **EUROPEAN PATENT APPLICATION**

(43) Date of publication:
**30.04.2008 Bulletin 2008/18**

(21) Application number: **06122740.1**

(22) Date of filing: **23.10.2006**

(51) Int Cl.:
***A61K 8/49*** *(2006.01)* ***A61Q 5/06*** *(2006.01)*
***A61Q 5/10*** *(2006.01)* ***C09B 29/00*** *(2006.01)*

(84) Designated Contracting States:
**AT BE BG CH CY CZ DE DK EE ES FI FR GB GR HU IE IS IT LI LT LU LV MC NL PL PT RO SE SI SK TR**
Designated Extension States:
**AL BA HR MK RS**

(71) Applicant: **Wella Aktiengesellschaft**
**64295 Darmstadt (DE)**

(72) Inventors:
- **Speckbacher, Markus**
**63739 Aschaffenburg (DE)**
- **Chassot, Jessica**
**1694 Chavannes-sous-Orsonnens (CH)**
- **Braun, Hans-Jürgen**
**3182 Ueberstorf (CH)**

(74) Representative: **Bockhorst, Matthias**
**Wella AG**
**Abteilung RP**
**Berliner Allee 65**
**D-64274 Darmstadt (DE)**

# (54) Dark coloured azo dyes

(57) The present invention relates to
d) a non-oxidative colouring agent for keratin fibres, in particular human hair, comprising at least one dye according to formula (I) or (II),
e) an oxidative colouring agent for keratin fibres, in particular human hair, which comprises at least one oxidizing agent (e.g. hydrogen peroxide), at least one oxidative dyc precursor and at least one dyc according to formula (I) or (II), and
f) a lightening agent for keratin fibres, in particular human hair, which comprises at least one oxidizing agent (e.g. hydrogen peroxide) and at least one dye according to formula (I) or (II), with R4 being a cationic group.

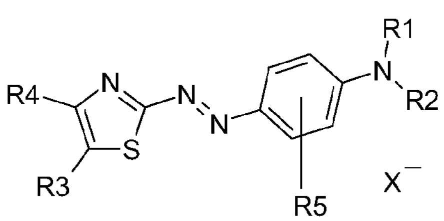

(I)

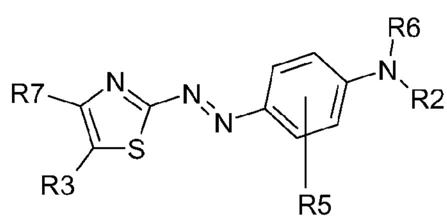

(II)

EP 1 915 984 A1

## Description

FIELD OF THE INVENTION

The present invention relates to dark coloured azo dyes, preparation methods for these azo dyes and formulations for dyeing and/or lightening keratin fibres, such as, for example, human hair, wool or furs, comprising these compounds.

BACKGROUND OF THE INVENTION

In general, two processes are used for colouring keratin-containing fibres. A first method relates to the well known oxidation dyes which are formed by oxidative coupling of one or more developer components (primary intermediates) with one or more coupler components. When using oxidizing agents, however, damage to the hair structure is observed. Furthermore, some of the oxidation hair colour precursors used may have a sensitization potential. If required, oxidation-stable direct dyes can also be added to the oxidative system in order to achieve particular colour effects. Another possibility consists in the exclusive use of direct dyes. These direct dyes are used in a suitable carrier masses. This method, generally known as tinting, is easy to use, exceptionally mild and is characterized by low damage to the keratin fibres since no ammonia or peroxide is added. These dyes should meet at least some basic requirements such as durability, washing and light fastness, good toxicological and dermatological profiles and, if desired, stability towards oxidizing agents (e.g. hydrogen peroxide). A colouration system based on direct dyes often requires combinations of several dyes to obtain the desired variety of different shades. This fact is of particular interest in the case for dark colours like black shades.

Normally, black shades can only be achieved by a mixture of several different direct dyes with different dyeing properties (e.g. diffusion etc.). It therefore would be desirable to have only one single black direct dye instead of a mixture of diverse direct dyes.

Griffith and Riepl (Griffith/Riepl, Chem. Commun. 1998, pp 1349-1350) published a promising route towards black-bluish monoazo disperse dyes and their use in liquid crystal displays and optical filters. The publication in Dyes and Pigments, Vol. 27, No.1, pp 45-54 (1995) and Dyes and Pigments, Vol. 37, No.4, pp 283-289 (1998) disclose special 4-amino-2-arylazothiazoles which shall have a good stability and shall be superior in some cases both thermally and photochemically, relativ to standard azo dyes; hair dyes are not disclosed. WO 2005/117816 A1 discloses cationic quinoxaline thiazole azo dyes and their use in colorants for keratin fibers. DE 102004056403 A1 discloses cationic pyridinylthiazo dyes and their use in colorants for keratin fibers. DE 102004051072 A1 discloses cationic heteroarylpyrazolonazo dyes and their use in colorants for keratin fibers.

SUMMARY OF THE INVENTION

An objective of the present invention is therefore to provide new dark coloured, respectively black direct dyes which are compatible to water based formulations and comply in addition to the abovementioned requirements.

Surprisingly, it has now been found that special cationic as well as anionic or neutral (uncharged) azo arrangements provide dark colours, can be applied gently to the fibres and produce naturally coloured shades. Since these dyes are also stable towards oxidizing agents (e.g. hydrogen peroxide), they can be used as well in lightening formulations or as supplement dyes in the oxidative system.

DETAILED DESCRIPTION OF THE INVENTION

Hence, the present invention relates to

a) an agent for the non-oxidative colouring of keratin fibres, in particular human hair, containing at least one dye according to formula (I) or (II),

b) an agent for the oxidative colouring of keratin fibres, in particular human hair, which comprises at least one oxidizing agent (e.g. hydrogen peroxide), at least one oxidative dye precursor and at least one dye according to formula (I) or (II), and

c) an agent for the lightening of keratin fibres, in particular human hair, which comprises at least one oxidizing agent (e.g. hydrogen peroxide) and at least one dye according to formula (I) or (II),

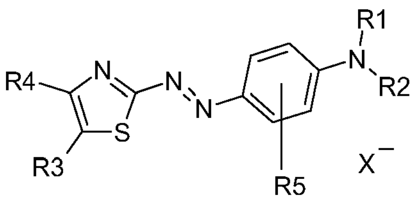

(I)

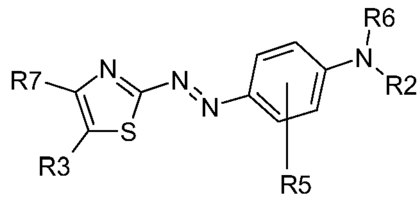

(II)

in which **R1** and **R2** may be identical or different and, independently of one another, are hydrogen, a benzyl group, a $C_1$-$C_6$-alkyl group, a $C_2$-$C_4$-hydroxyalkyl group, a $C_2$-$C_4$-cyanoalkyl group or a $C_4$-$C_6$-polyhydroxyalkyl group, wherein the alkyl groups can be both branched and linear; and
**R3** is a group according to the general formulas (III), (IV), (V), (VI) or (VII),

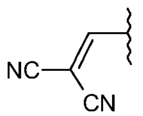

(III)

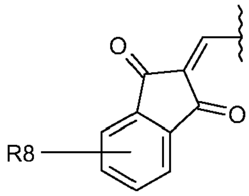

(IV)

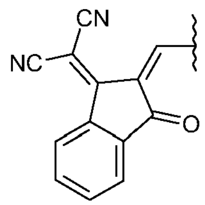

(V)

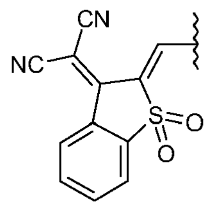

(VI)

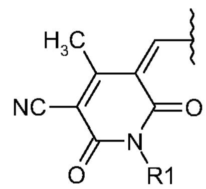

(VII)

or a formyl group, in which **R8** is a hydrogen, a halogen atom, a $C_1$-$C_6$-alkyl group, or a nitro group and in which **R1** has the abovementioned meaning; and
**R4** is a cationic group according to the general formula (VIII) or (IX),

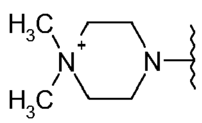

(VIII)

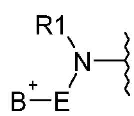

(IX)

in which **E** may be a $C_1$-$C_4$-alkyl group, a $C_3$-$C_6$-cycloalkyl group or an arylgroup; and **R1** has the above-mentioned meaning, and **B**$^+$ may be

a) a cationic, aromatic heterocyclic ammonium compound, preferably a cationic derivative of N-methyl-imidazole, N-allyl-imidazole, 2-ethyl-imidazole or 1,2-dimethyl-imidazole or a cationic derivative of pyridine, 4-dimethylamino-pyridine, pyrimidine, pyrazole, N- methylpyrazole or chinoline; or
b) a non-aromatic heterocyclic ammonium compound, in particular a cationic derivative of N-methyl-morpholine, N-ethyl-morpholine or 1-methyl-piperidine; or
c) a cationic alkylammonium compound or an arylammonium compound according to the formula $NR_aR_bR_c$, in which $\mathbf{R_a}$, $\mathbf{R_b}$ and $\mathbf{R_c}$, independently of each other, are a benzyl rest, a phenyl rest or a $C_1$-$C_6$-alkyl rest, preferably a methyl group, an ethyl group, a propyl group, an isopropyl group or a butyl group, whereas the prementioned alkyl groups may be unsubstituted or substituted with one or more hydroxy groups or amino groups; or
d) a cationic phosphonium group, e.g. a tributyl phosphonium group, preferably a trimethyl phosphonium group or a triethyl phosphonium group; and

**R5** is hydrogen, a halogen atom, a $C_1$-$C_6$-alkyl group, a hydroxy group, a $C_1$-$C_6$-alkyloxy group, an amino group or an amino-acetyl group; and
**R6** is a $C_1$-$C_6$-alkylsulfonic acid group, a $C_1$-$C_6$-alkylsulfate group (a $C_1$-$C_6$-alkylsulfonic acid ester) or their corresponding sodium or potassium salts, respectively; and
**R7** is a chloro atom, a pyrrolidine rest, a piperidine rest, or a *N*-methyl-piperazine rest or an aliphatic secondary amine rest bearing two $C_1$-$C_4$-alkyl chains which may be identical or different; and
X- is a anionic counterion, preferably a sulphate anion, a methylsulphate anion, a phosphate anion, a hydrogenphosphate anion, an oxalate anion, a formate anion, an acetate anion, a citrate anion, a tartrate anion, a malonate anion, a pyruvate anion or a halogen anion, particular preference being given to the methylsulphate anion.

The dyes according to the general formula (I) or (II) are preferable chosen from

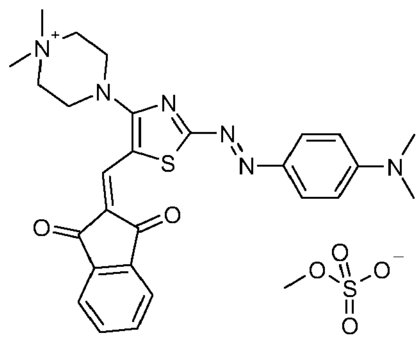

4-(2- {(*E*)-[4-(dimethylamino)phenyl]diazenyl}-5-[(1,3-dioxo-1,3-dihydro-2*H*-inden-2-ylidene)methyl]-1,3-thiazol-4-yl)-1,1-dimethylpiperazin-1-ium methyl sulfate (1)

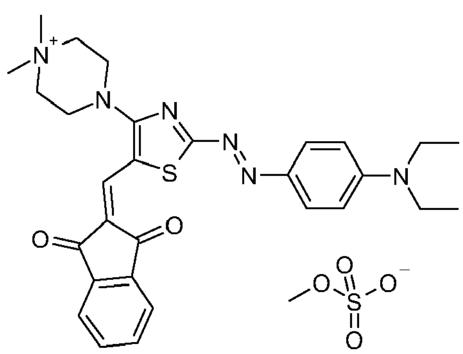

4-{2-{(*E*)-[4-(diethylamino)phenyl]diazenyl}-5-[(1,3-dioxo-1,3-dihydro-2*H*-inden-2-ylidene)methyl]-1,3-thiazol-4-yl}-1,1-dimethylpiperazin-1-ium methyl sulfate (2)

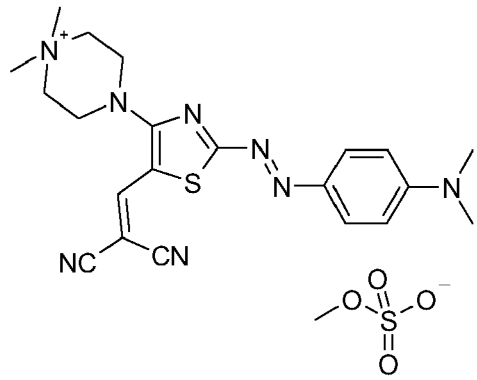

4-(5-(2,2-dicyanovinyl)-2- {(*E*)-[4-(dimethylamino)phenyl]diazenyl}-1,3-thiazol-4-yl)-1,1-dimethylpiperazin-1-ium methyl sulfate **(3)**

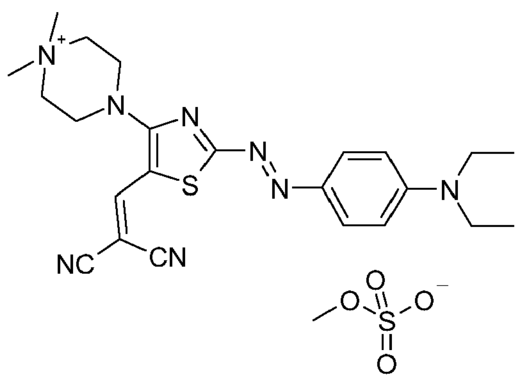

4-(5-(2,2-dicyanovinyl)-2- {(*E*)-[4-(diethylamino)phenyl]diazenyl}-1,3-thiazol-4-yl)-1,1-dimethylpiperazin-1-ium methyl sulfate **(4)**

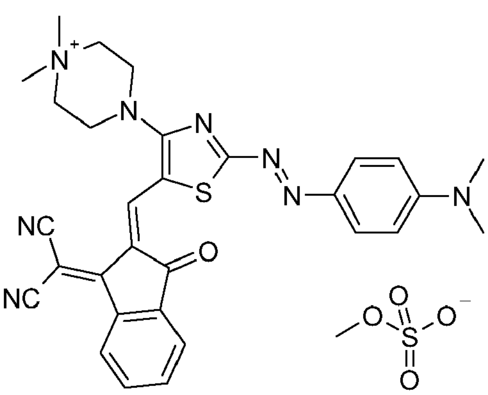

4-(5- {(*Z*)-[1-(dicyanomethylene)-3-oxo-1,3-dihydro-2*H*-inden-2-ylidene]methyl}-2-{(*E*)-[4-(dimethylamino)phenyl]diazenyl}-1,3-thiazol-4-yl)-1,1-dimethylpiperazin-1-ium methyl sulfate (5)

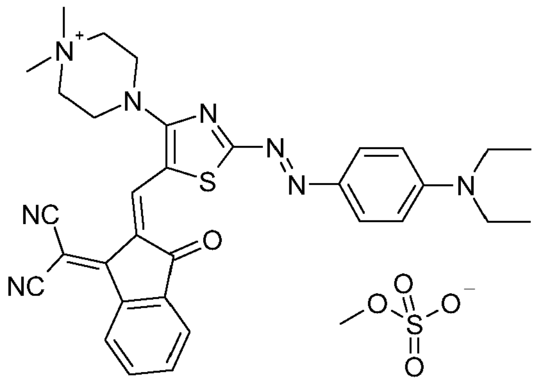

4-(5- {(*Z*)-[1-(dicyanomethylene)-3-oxo-1,3-dihydro-2*H*-inden-2-ylidene]methyl}-2-{(*E*)-[4-(diethylamino)phenyl]diazenyl}-1,3-thiazol-4-yl)-1,1-dimethylpiperazin-1-ium methylsulfate (6)

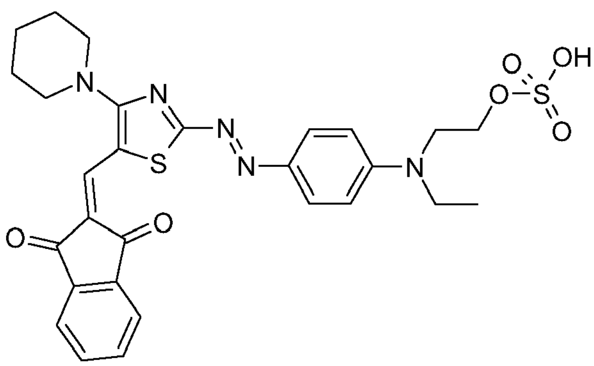

2-[{4-[(*E*)-{5-[(1,3-dioxo-1,3-dihydro-2*H*-inden-2-ylidene)methyl]-4-(1-piperidinyl)-1,3-thiazol-2-yl}diazenyl]phenyl}(ethyl)amino]ethyl hydrogen sulfate (7)

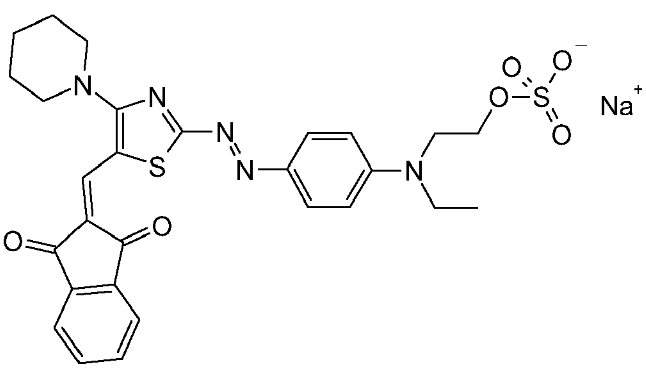

sodium 2-[{4-[(*E*)-{5-[(1,3-dioxo-1,3-dihydro-2*H*-inden-2-ylidene)methyl]-4-(1-piperidinyl)-1,3-thiazol-2-yl}diazenyl]phenyl}(ethyl)amino]ethyl sulfate **(8)**

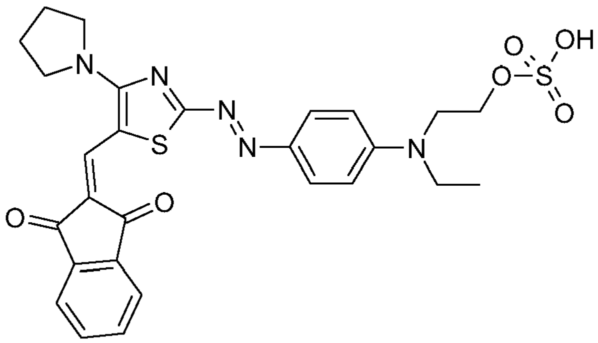

2-[{4-[(*E*)-{5-[(1,3-dioxo-1,3-dihydro-2*H*-inden-2-ylidene)methyl]-4-(1-pyrrolidinyl)-1,3-thiazol-2-yl}diazenyl]phenyl}(ethyl)amino]ethyl hydrogen sulfate (**9**)

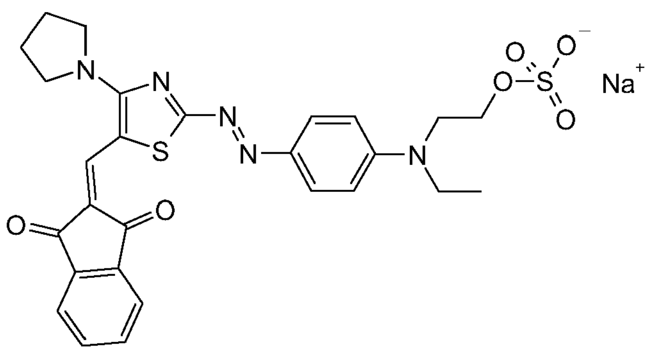

sodium 2-[{4-[(*E*)-{5-[(1,3-dioxo-1,3-dihydro-2*H*-inden-2-ylidene)methyl]-4-(1-pyrrolidinyl)-1,3-thiazol-2-yl}diazenyl]phenyl}(ethyl)amino]ethyl sulfate **(10)**

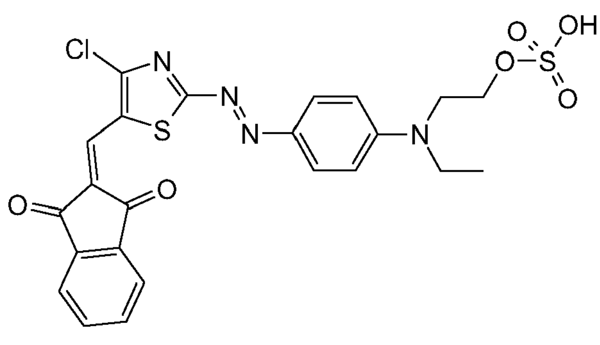

2-[ {4-[(*E*)-{4-chloro-5-[(1,3-dioxo-1,3-dihydro-2*H*-inden-2-ylidene)methyl]-1,3-thiazo1-2-yl}diazenyl]phenyl}(ethyl)amino]ethyl hydrogen sulfate **(11)**

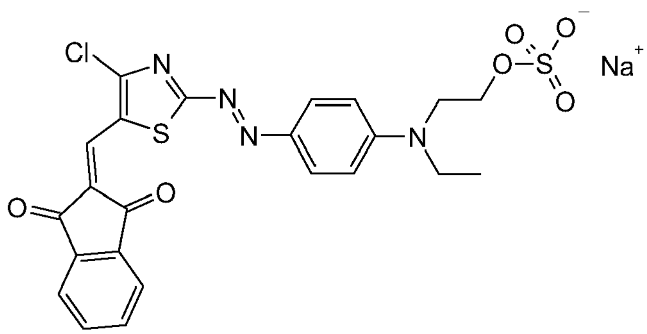

sodium 2-[{4-[(*E*)-{4-chloro-5-[(1,3-dioxo-1,3-dihydro-2*H*-inden-2-ylidene)methyl]-1,3-thiazol-2-yl}diazenyl]phenyl}(ethyl)amino]ethyl sulfate **(12)**

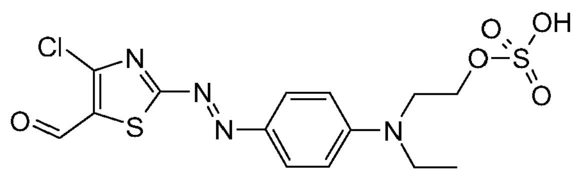

2-[{4-[(*E*)-(4-chloro-5-formyl-1,3-thiazol-2-yl)diazenyl]phenyl} (ethyl)amino]ethyl hydrogen sulfate **(13)**

Particularly preferred are: 4-{2-{(*E*)-[4-(dimethylamino)phenyl]diazenyl}-5-[(1,3-dioxo-1,3-dihydro-2*H*-inden-2-ylidene)methyl]-1,3-thiazol-4-yl}-1,1-dimethylpiperazin-1-ium methylsulfate, 4- {2-{(*E*)-[4-(diethylamino)phenyl]diazenyl}-5-[(1,3-dioxo-1,3-dihydro-2*H*-inden-2-ylidene)methyl]-1,3-thiazol-4-yl}-1,1-dimethylpiperazin-1-ium methylsulfate, 2-[{4-[(*E*)- {5-[(1,3-dioxo-1,3-dihydro-2*H*-inden-2-ylidene)methyl]-4-(1-piperidinyl)-1,3-thiazol-2-yl}diazenyl]phenyl}(ethyl)amino]ethyl hydrogen sulfate, 2-[{4-[(*E*)-{5-[(1,3-dioxo-1,3-dihydro-2*H*-inden-2-ylidene)methyl]-4-(1-pyrrolidinyl)-1,3-thiazol-2-yl}diazenyl]phenyl}(ethyl)amino]ethyl hydrogen sulfate, 2-[{4-[(*E*)-{4-chloro-5-[(1,3-dioxo-1,3-dihydro-2*H*-inden-2-ylidene)methyl]-1,3-thiazol-2-yl}diazenyl]phenyl}(ethyl)amino]ethyl hydrogen sulfate sulfate and 2-[{4-[(*E*)-(4-chloro-5-formyl-1,3-thiazol-2-yl)diazenyl]phenyl}(ethyl)amino]ethyl hydrogen sulfate.

The dyes according to the invention of the general formula (I) and (II) can be prepared via a multi-step synthesis starting with diazotation of 2-amino-4-chloro-1,3-thiazole-5-carbaldehyde (EP 0450438 A1 and Kasali A. Bello, Dyes and Pigments, Vol. 27, 1995, No. 1, 45-54) and subsequent coupling with various substituted anilines to obtain first intermediate dye derivatives, represented by structure **A**, in which $\mathbf{R_1}$ and $\mathbf{R_2}$ have the abovementioned meaning (scheme 1).

Scheme 1:

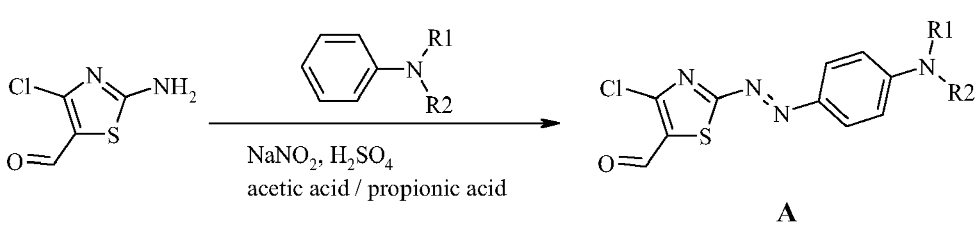


**A**

In a second step, an appropriate CH-acide acceptor-group (e.g. 1*H*-indene-1,3(2*H*)-dione) was condensed with the thiazole ring in an alcoholic medium to yield compounds according to structure **B** (scheme 2). This operation was performed in accordance with literature procedures (Masaki Matsui et al., Dyes and Pigments, Vol. 37, 1998, No. 4, 283-289).

Scheme 2:

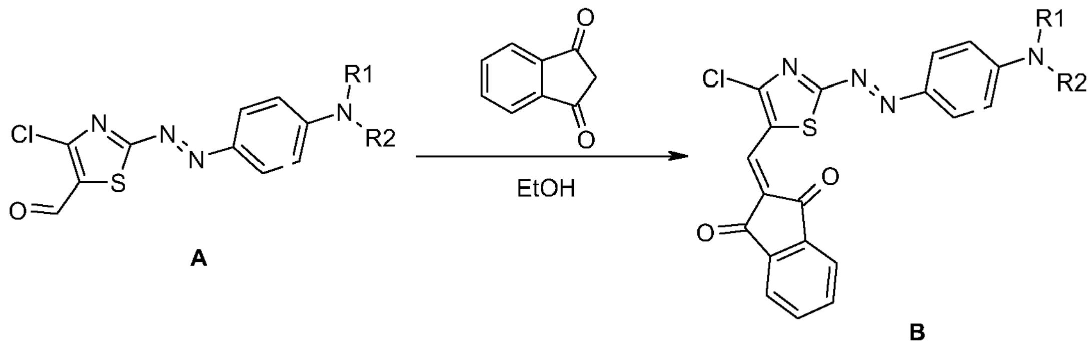


The third step involves a nucleophilic substitution of the chloro atom of **B** by secondary amines such as piperidine (John Griffith et al., Chem. Commun., 1998, 1349-1350) or 1-methylpiperazine in solvents like THF (tetrahydrofurane) to obtain dark coloured dyes according to structure **C** (scheme 3).

Scheme 3:

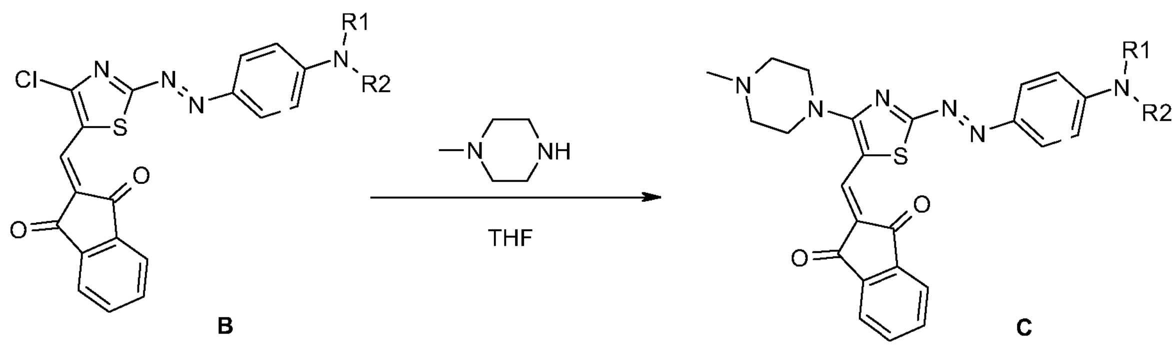


The final step, treatment of **C** with alkylating agents like dimethylsulfate in acetonitrile, results in the formation of desired cationic dyes (shown as **D**) according to the invention (scheme 4).

Scheme 4:

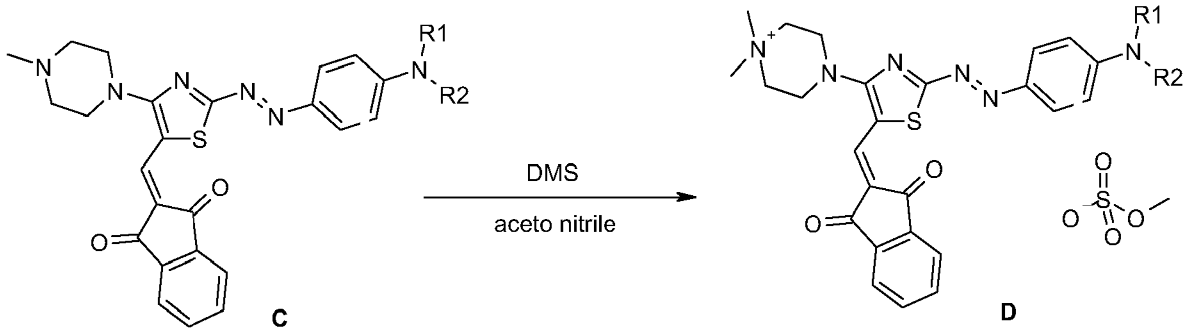


C

D

Anionic derivatives are accesible by reacting chloro sulfonic acid with an appropriate hydroxyl anchor to yield the corresponding sulfate (sulfonic acid ester) followed by conversion into an alkalimetal (e.g. sodium) salt according to the invention, if desired. However, in most cases the uncharged sulfate showed sufficient solubility. An example (designated with E, respectively **E***) is illustrated in scheme 5.

Scheme 5:

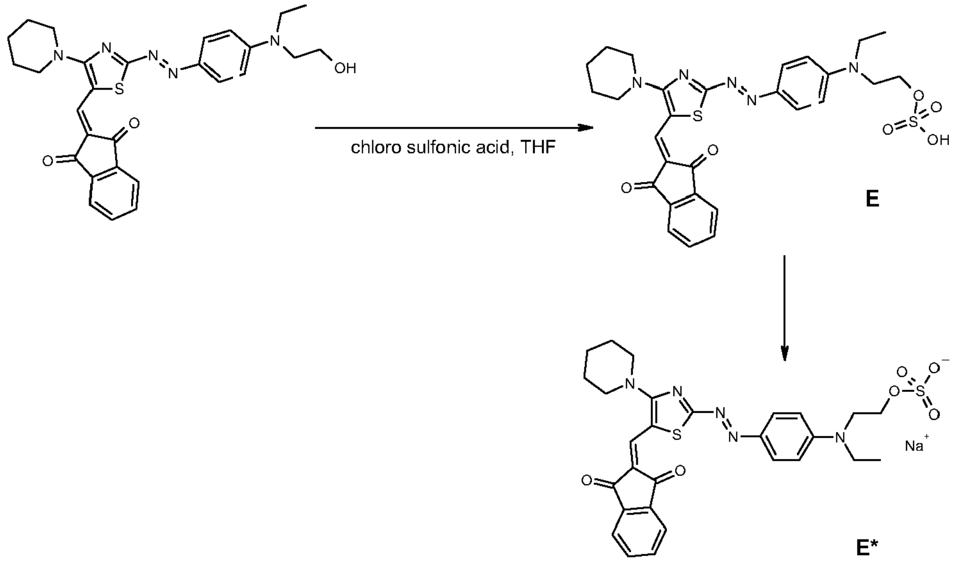


E

E*

Formulations, containing dyes according to the invention of the general formula (I) or (II) provide an even colouration on keratin fibres, in particular human hair, with favorable dyeing properties such as good fastness to light, washing, rubbing and perspiration. Natural colourations with low selectivity can be obtained already under gentle conditions.

The dyes of the general formula (I) or (II) according to the invention are preferably present in the colourants according to the invention in a total amount of from 0.01 to 10 percent by weight, in particular 0.1 to 8 percent by weight.

To produce special colour shades, besides the dyes of the general formula (I) or (II) according to the invention, it is possible to add to the agents according to the invention one or more additional customary direct dye from the group consisting of acidic dyes, basic dyes, nitro dyes, azo dyes, anthraquinone dyes and triphenylmethane dyes, such as for example, 1,4-bis[(2-hydroxyethyl)amino]-2-nitrobenzene, 1-(2-hydroxyethyl)amino-2-nitro-4-[di-(2-hydroxyethyl)amino]benzene, (HC Blue No. 2), 1-amino-3-methyl-4-[(2-hydroxyethyl)amino]-6-nitrobenzene, (HC Violet No. 1), 4-[ethyl-(2-hydroxyethyl)amino]-1-[(2-hydroxyethyl)amino]-2-nitrobenzene hydrochloride (HC Blue No. 12), 1-[(2,3-dihydroxypropyl)amino]-4-[methyl-(2-hydroxyethyl)amino]-2-nitrobenzene (HC Blue No. 10), 1-[(2,3-dihydroxypropyl)amino]-4-[ethyl-(2-hydroxyethyl)amino]-2-nitrobenzene hydrochloride (HC Blue No. 9), 1-(3-hydroxypropylamino)-4-[di(2-hydroxyethyl)amino]-2-nitrobenzene, (HC Violet No. 2); 1-amino-4-[(2-hydroxyethyl)amino]-2-nitrobenzene (HC Red No. 7), 2-amino-4,6-dinitrophenol, 1,4-diamino-2-nitrobenzene (CI76070), 4-amino-2-nitrodiphenylamine (HC Red No. 1), 1-amino-4-[di(2-hydroxyethyl)amino]-2-nitrobenzene hydrochloride (HC Red No. 13), 1 -amino-5-chloro-4-[(2-hydroxyethyl)amino]-2-nitrobenzene, 4-amino-1-[(2-hydroxyethyl)-amino]-2-nitrobenzene (HC Red No. 3), 4-amino-2-nitro-1-((prop-2-en-1-yl)amino)benzene, 4-amino-3-nitrophenol, 4-[(2-hydroxyethyl)amino]-3-nitrophenol, 4-[(2-nitrophenyl)amino]phenol (HC Orange No. 1), 1-[(2-aminoethyl)amino]-4-(2-hydroxyethoxy)-2-nitrobenzene (HC Orange No. 2), 4-(2,3-dihydroxypropoxy)-1-[(2-hydroxyethyl)amino]-2-nitrobenzene, (HC Orange No. 3), 1-amino-5-chloro-4-[(2,3-dihydroxypropyl)amino]-2-nitrobenzene (HC Red No. 10), 5-chloro-1,4-[di(2,3-dihydroxypropyl)amino]-2-nitrobenzene (HC Red No. 11), 2-[(2-hydroxyethyl)amino]-4,6-dinitrophenol, 4-ethylamino-3-nitrobenzoic acid, 2-[(4-amino-2-nitrophenyl)amino]benzoic acid, 2-chloro-6-ethylamino-4-nitrophenol, 2-amino-6-chloro-4-nitrophenol, 4-[(3-hydroxypropyl)amino]-3-nitrophenol, 2,5-diamino-6-nitropyridine, 3-amino-6-(methylamino)-2-nitropyridine, 1,2,3,4-tetrahydro-6-nitroquinoxaline, 7-amino-3,4-dihydro-6-nitro-2H-1,4-benzoxazine (HC Red No. 14), 1,2-diamino-4-nitrobenzene (CI76020), 1-amino-2-[(2-hydroxyethyl)amino]-5-nitrobenzene (HC Yellow No. 5), 1-(2-hydroxyethoxy)-2-[(2-hydroxyethyl)amino]-5-nitrobenzene, (HC Yellow No. 4), 1-[(2-hydroxyethyl)amino]-2-nitrobenzene (HC Yellow No. 2), 2-[(2-hydroxyethyl)amino]-1-methoxy-5-nitrobenzene, 2-amino-3-nitrophenol, 1-amino-2-methyl-6-nitrobenzene, 1-(2-hydroxyethoxy)-3-methylamino-4-nitrobenzene, 2,3-(dihydroxypropoxy)-3-methylamino-4-nitrobenzene, 2-[(2-hydroxyethyl)amino]-5-nitrophenol (HC Yellow No. 11), 3-[(2-aminoethyl)amino]-1-methoxy-4-nitrobenzene hydrochloride, (HC Yellow No. 9), 1-[(2-ureidoethyl)amino]-4-nitrobenzene, 4-[(2,3-dihydroxypropyl)amino]-3-nitro-1-trifluoromethylbenzene, (HC Yellow No. 6), 1-chloro-2,4-bis[(2-hydroxyethyl)amino]-5-nitrobenzene (HC Yellow No. 10), 4-[(2-hydroxyethyl)amino]-3-nitro-1-methylbenzene, 1-chloro-4-[(2-hydroxyethyl)amino]-3-nitrobenzene (HC Yellow No. 12), 4-[(2-hydroxyethyl)amino]-3-nitro-1-trifluoromethylbenzene, (HC Yellow No. 13), 4-[(2-hydroxyethyl)amino]-3-nitrobenzonitrile (HC Yellow No. 14), 4-[(2-hydroxyethyl)amino]-3-nitrobenzamide (HC Yellow No. 15), 2,4-dinitro-1-hydroxynaphthalene, 1,4-di[(2,3-dihydroxypropyl)amino]-9,10-anthraquinone, 1,4-di[(2-hydroxyethyl)amino]-9,10-anthraquinone (CI61545, Disperse Blue 23), 1-amino-4-hydroxy-9,10-anthraquinone (CI60710, Disperse Red 15), 1-hydroxy-4-[(4-methyl-2-sulphophenyl)-amino]-9,10-anthraquinone, 7-beta-D-glucopyranosyl-9,10-dihydro-1-methyl-9,10-dioxo-3,5,6,8-tetrahydroxy-2-anthracenecarboxylic acid (CI75470, Natural Red 4), 1-[(3-aminopropyl)amino]-9,10-anthraquinone (HC Red No. 8), 1,4-diamino-9,10-anthraquinone (CI61100, Disperse Violet No. 1), 1-amino-4-(methylamino)-9,10-anthraquinone (CI61105, Disperse Violet No. 4, Solvent Violet No. 12), N-(6-((3-chloro-4-(methylamino)phenyl)imino)-4-methyl-3-oxo-1,4-cyclohexadien-1-yl)urea (HC Red No. 9), 2-((4-(di(2-hydroxyethyl)amino)phenyl)amino)-5-((2-hydroxyethyl)-amino)-2,5-cyclohexadiene-1,4-dione (HC Green No. 1), 2-hydroxy-1,4-naphthoquinone (CI75480, Natural Orange No. 6), 1,2-dihydro-2-(1,3-dihydro-3-oxo-2H-indol-2-ylidene)-3H-indol-3-one (CI73000), 1,3-bis(dicyanomethylene)indane, di[4-(diethylamino)phenyl]-[4-(ethylamino)naphthyl]carbenium chloride (CI42595; Basic Blue No. 7), di[4-(dimethylamino)phenyl][4-(phenylamino)naphthyl]carbenium chloride (CI44045; Basic Blue No. 26), Basic Blue No. 77, 8-amino-2-bromo-5-hydroxy-4-imino-6-[(3-(trimethylammonio)phenyl)amino]-1(4H)-naphthalinone chloride (CI56059; Basic Blue No. 99), tri(4-amino-3-methylphenyl)carbenium chloride (CI42520; Basic Violet No. 2), di(4-aminophenyl)(4-amino-3-methylphenyl)carbenium chloride (CI42510; Basic Violet No. 14), 1-[(4-aminophenyl)azo]-7-(trimethylammonio)-2-naphthol chloride (CI12250; Basic Brown No. 16), 3-[(4-amino-2,5-dimethoxyphenyl)azo]-N,N,N-trimethylbenzenaminium chloride (CI112605, Basic Orange No. 69), 1-[(4-amino-2-nitrophenyl)azo]-7-(trimethylammonio)-2-naphthol chloride (Basic Brown No. 17), 1-[(4-amino-3-nitrophenyl)azo]-7-(trimethylammonio)-2-naphthol chloride (CI12251; Basic Brown No. 17), 2-((4-aminophenyl)azo)-1,3-dimethyl-1H-imidazol-3-ium chloride (Basic Orange No. 31), 3,7-diamino-2,8-dimethyl-5-phenylphenazinium chloride (CI50240; Basic Red No. 2), 1,4-dimethyl-5-[(4-(dimethylamino)phenyl)azo]-1,2,4-triazolium chloride (CI11055; Basic Red No. 22), 1,3-dimethyl-2-((4-dimethylamino)phenyl)azo-1H-imidazol-3-ium chloride (Basic Red No. 51), 2-hydroxy-1-[(2-methoxyphenyl)azo]-7-(trimethylammonio)naphthalene chloride (CI12245; Basic Red No. 76), 3-methyl-1-phenyl-4-[(3-(trimethylammonio)phenyl)azo]-pyrazol-5-one chloride (CI12719; Basic Yellow No. 57), 1-methyl-4-((methylphenyl-hydrazono)methyl)pyridinium methylsulphate (Basic Yellow No. 87), 1-(2-morpholiniumpropylamino)-4-hydroxy-9,10-anthraquinone methylsulphate, 1-[(3-(dimethylpropylaminium)propyl)amino]-4-(methylamino)-9,10-anthraquinone chloride, 1-[di(2-hydroxyethyl)amino]-3-methyl-4-[(4-nitrophenyl)azo]benzene (CI11210, Disperse Red No. 17), 1-[di(2-hydroxyethyl)amino]-4-[(4-nitrophenyl)azo]benzene, (Disperse Black No. 9), 4-[(4-aminophenyl)azo]-1-[di(2-hydroxyethyl)amino]-3-methylbenzene, (HC Yellow No. 7), 2,6-diamino-3-[(pyridin-3-yl)azo]-pyridine and 2-((4-(ethyl(2-hydroxyethyl)amino)-2-methylphenyl)azo)-5-nitro-1,3-thiazole (CI111935; Disperse Blue No. 106), 3-(2',6'-diamino-pyridyl-3'-azo)pyridine (= 2,6-diamino-3-((pyridin-3-yl)azo)pyridine, N,N-di(2-hydroxyethyl)-3-methyl-4-((4-nitrophenyl)azo)aniline (Disperse Red 17, CI 11210), 3-diethylamino-7-(4-dimethylaminophenylazo)-5-phenylphenazinium chloride (CI 11050), 4-(2-thiazolylazo)resorcinol, 4-((4-phenylamino)azo)benzosulphonic acid sodium salt (Orange IV), 1-((3-aminopropyl)amino)-9,10-anthracenedione, (HC Red No. 8), 3',3",4,5,5',5",6,7-octabromophenol sulphonephthalein (Tetrabromophenol Blue), 1-((4-amino-3,5-dimethylphenyl)-(2,6-dichlorophenyl)methylene)-3,5-dimethyl-4-imino-2,5-cyclohexadiene phosphoric acid (1:1) (Basic Blue 77), 3',3",5',5"-tetrabromo-m-cresol sulphonephthalein, 2,4-dinitro-1-naphthol-7-sulphonic acid disodium salt (Acid Yellow 1, CI 10316), 4-[2'-hydroxy-1'-naphthyl)azo]benzosulphonic acid sodium salt (Acid Orange 7, CI 15510), 3',6'-dihydroxy-2',4',5',7'-tetraiodospiro-[isobenzofuran-1(3H), 9'(9H)-xanthen]-3-one disodium salt (Acid Red 51, CI 45430), 6-hydroxy-5-((2-methoxy-5-methyl-4-sulphophenyl)azo)-2-naphthalenesulphonic acid disodium salt (FD&C Red 40, CI 16035), 2,4-dinitro-1-naphthol sodium salt (Acid Yellow 24; CI 10315), 2',4',5',7'-tetrabromo-4,5,6,7-tetrachloro-3',6'-dihydroxyspiro-(isobenzofuran-1(3H), 9'[9H]xanthen]-3-one disodium salt (Acid Red 92; CI 45410), 4-(2-hydroxy-1-naphthylazo)-3-methyl-benzenesulphonic acid sodium salt (Acid Orange 8, CI 15575), 2-amino-1,4-naphthalenedione, dithizone (1,5-diphenylthiocarbazone), N-(2-hydroxyethyl))-2-nitro-4-trifluoromethyl)aniline (HC Yellow 13), N-(2-hydroxyethyl)-4-nitroaniline and 4-chloro-N-(2,3-dihydroxypropyl)-2-nitroaniline.

In addition, the colourants according to the invention can also comprise naturally occurring dyes, such as, for example, henna red, henna neutral, henna black, camomile, sandalwood, black tea, buckthorn bark, sage, logwood, madder root, catechu, sedre and alkanna root.

The abovementioned additional naturally occuring dyes and direct dyes may be present in a total amount of from about 0.01 to 5 percent by weight, the total content of dyes in the colourant according to the invention being preferably from about 0.01 to 10 percent by weight, in particular 0.1 to 5 percent by weight.

The oxidative colouring system according to the invention, which is mixed with hydrogen peroxide as oxidizing agent prior to application, contains beside direct dyes according to formulas (I) and (II), at least one or more oxidative dye precursors. Suitable oxidative dye precursers (primary intermediates and coupler substances) are defined as follows:

(i) primary intermediates: 1,4-diaminobenzen (p-phenylenediamine), 1,4-diamino-2-methylbenzene (p-toluylenediamine), 1,4-diamino-2,6-dimethylbenzene, 1,4-diamino-3,5-diethylbenzene, 1,4-diamino-2,5-dimethylbenzene, 1,4-diamino-2,3-dimethylbenzene, 2-chloro-1,4-diaminobenzene, 1,4-diamino-2-(thiophen-2-yl)benzene, 1,4-diamino-2-(thiophen-3-yl)benzene, 2-(6-2,5-methyl-pyridin-2-yl)-benzene-1,4-diamine, 2-thiazol-2-yl-benzene-1,4-diamino, 1,4-diamino-2-(pyridin-3-yl)benzene, 2,5-diaminobiphenyl, 2,5-diamino-4'-(1-methylethyl)-1,1'-biphenyl, 2,3',5-triamino-1,1'-biphenyl, 2'-chloro-1,1'-biphenyl-2,5-diamine, 3'-fluoro-1,1'-biphenyl-2,5-diamine, 1,4-diamino-2-methoxymethylbenzene, 1,4-diamino-2-aminomethylbenzene, 3-(3-amino-phenylamino-propenyl)-benzene-1,4-diamine, 2-propenylbenzene-1,4-diamine, 1,4-diamino-2-((phenylamino)methyl)-benzene, 1,4-diamino-2-((ethyl-(2-hydroxyethyl)-amino)methyl)benzene, 1,4-diamino-2-hydroxymethylbenzene, 1,4-diamino-2-(2-hydroxyethoxy)-benzene, 2-(2-(acetylamino)ethoxy)-1,4-diaminobenzene, 4-(phenyl-amino)aniline, 4-(dimethylamino)aniline, 4-(diethylamino)aniline, 4-(dipropylamino)aniline, 4-[ethyl(2-hydroxyethyl)amino]aniline, 4-[di(2-hydroxyethyl)amino]aniline, 4-[di(2-hydroxyethyl)amino]-2-methyl-aniline, 4-[(2-methoxyethyl)amino]aniline, 4-[(3-hydroxypropyl)amino]aniline, 4-[(2,3-dihydroxypropyl)amino]aniline, 4-(((4-aminophenyl)methyl)amino)aniline, 4-[(4-amino-phenylamino)-methyl]-phenol, 3-((4-amino-phenylamino)methyl)phenol, 1,4-diamino-N-(4-pyrrolidin-1-yl-benzyl)benzene, 1,4-diamino-N-furan-3-ylmethylbenzene, 1,4-diamino-N-thiophen-2-ylmethylbenzene, 1,4-diamino-N-furan-2-ylmethylbenzene, 1,4-diamino-N-thiophen-3-ylmethylbenzene, 1,4-diamino-N-benzylbenzene, 1,4-diamino-2-(1-hydroxyethyl)benzene, 1,4-diamino-2-(2-hydroxyethyl)benzene, 1,4-diamino-2-(1-methylethyl)benzene, 1,3-bis[(4-aminophenyl)(2-hydroxyethyl)amino]-2-propanol, 1,4-bis[(4-aminophenyl)amino]-butane, 1,8-bis(2,5-diaminophenoxy)-3,6-dioxaoctane, 2,5-diamino-4'-hydroxy-1,1'-biphenyl, 2,5-diamino-2'-trifluoromethyl-1,1'-biphenyl, 2,4',5-triamino-1,1'-biphenyl, 4-amino-phenol, 4-amino-3-methylphenol, 4-amino-3-(hydroxymethyl)-phenol, 4-amino-3-fluoro-phenol, 4-methylaminophenol, 4-amino-2-(aminomethyl)phenol, 4-amino-2-(hydroxymethyl)phenol, 4-amino-2-fluorophenol, 4-amino-2-[(2-hydroxyethyl)-amino]methyl-phenol, 4-amino-2-methyl-phenol, 4-amino-2-(methoxymethyl)phenol, 4-amino-2-(2-hydroxyethyl)phenol, bis(5-amino-2-hydroxyphenyl)phenol, 5-amino-salicylic acid, 2,5-diamino-pyridine, 2,4,5,6-tetraaminopyrimidine, 2,5,6-Triamino-4-(1H)-pyrimidone, 4,5-diamino-1-(2-hydroxyethyl)-1H-pyrazole, 4,5-diamino-1-(1-methylethyl)-1H-pyrazole, 4,5-diamino-1-[(4-methylphenyl)methyl]-1H-pyrazole, 1-[(4-chlorophenyl)methyl]-4,5-diamino-1H-pyrazole,4,5-diamino-1-methyl-1H-pyrazole, 4,5-diamino-1-pentyl-1H-pyrazole, 4,5-diamino-1-(phenylmethyl)-1H-pyrazole, 4,5-diamino-1-((4-methoxyphenyl)methyl-1H-pyrazole, 2-aminophenol, 2-amino-6-methylphenol, 2-amino-5-methyl-phenol, 4-amino-1,1'-biphenyl-3-ol, 2-amino-5-ethylphenol, 1,2,4-trihydroxybenzene, 2,4-diaminophenol, 1,4-dihydroxybenzene, 2-(((4-aminophenyl)-amino)methyl)-1,4-diaminobenzene.

(ii) coupler substances: N-((3-aimethylamino)phenyl)urea, 2,6-aiaminopyridine, 2-amino-4-[(2-hydroxyethyl)amino]-anisole, 2,4-diamino-1-fluoro-5-methylbenzene, 2,4-diamino-1-methoxy-5-methylbenzene, 2,4-diamino-1-ethoxy-5-methylbenzene, 2,4-diamino-1-(2-hydroxyethoxy)-5-methylbenzene, 2,4-di[(2-hydroxyethyl)amino]-1,5-dimethoxybenzene, 2,3-diamino-6-methoxypyridine, 3-amino-6-methoxy-2-(methylamino)pyridine, 2,6-diamino-3,5-dimethoxypyridine, 3,5-diamino-2,6-dimethoxypyridine, 1,3-diaminobenzene, 2,4-diamino-1-(2-hydroxyethoxy)benzene, 1,3-diamino-4-(2,3-dihydroxypropoxy)benzene, 1,3-diamino-4-(3-hydroxypropoxy)benzene, 1,3-diamino-4-(2-methoxyethoxy)benzene, 2,4-diamino-1,5-di(2-hydroxyethoxy)benzene, 1-(2-aminoethoxy)-2,4-diaminobenzene, 2-amino-1-(2-hydroxyethoxy)-4-methylaminobenzene, 2,4-diaminophenoxyacetic acid, 3-[di(2-hydroxyethyl) amino]aniline, 4-amino-2-di[(2-hydroxyethyl)amino]-1-ethoxybenzene, 5-methyl-2-(1-methylethyl)phenol, 3-[(2-hydroxyethyl)amino]aniline, 3-[(2-aminoethyl)amino]aniline, 1,3-di(2,4-diaminophenoxy)propane, di(2,4-diaminophenoxy)methane, 1,3-diamino-2,4-dimethoxybenzene, 2,6-bis(2-hydroxyethyl)amino-toluene, 4-hydroxyindole, 3-dimethylaminophenol, 3-(diethylamino)phenol, 5-amino-2-methylphenol, 5-amino-4-fluoro-2-methylphenol, 5-amino-4-methoxy-2-methylphenol, 5-amino-4-ethoxy-2-methylphenol, 3-amino-2,4-dichlorophenol, 5-amino-2,4-dichlorophenol, 3-amino-2-methylphenol, 3-amino-2-chloro-6-methylphenol, 3-amino-2,6-dimethylphenol, 3-aminophenol, 2-[(3-hydroxyphenyl)amino]acetamide, 5-[(2-hydroxyethyl)amino]-4-methoxy-2-methylphenol, 5-[(2-hydroxyethyl)-amino]-2-methylphenol, 3-[(2-hydroxyethyl)amino]phenol, 3-[(2-methoxyethyl)amino]phenol, 5-amino-2-ethylphenol, 5-amino-2-methoxyphenol, 2-(4-amino-2-hydroxyphenoxy)ethanol, 5-[(3-hydroxypropyl)amino]-2-methylphenol, 3-[(2,3-dihydroxypropyl)amino]-2-methylphenol, 3-[(2-hydroxyethyl)amino]-2-methylphenol, 2-amino-3-hydroxypyridine, 2,6-dihydroxy-3,4-dimethylpyridine, 5-amino-4-chloro-2-methylphenol, 1-naphthol, 2-methyl-1-naphthol, 1,5-dihydroxynaphthalene, 1,7-dihydroxynaphthalene, 2,3-dihydroxynaphthalene, 2,7-dihydroxy-naphthalene, 2-methyl-1-naphthol-acetate, 1,3-dihydroxybenzene, 1-chloro-2,4-dihydroxybenzene, 2-chloro-1,3-dihydroxybenzene, 1,2-dichloro-3,5-dihydroxy-4-methylbenzene, 1,5-dichloro-2,4-di-hydroxybenzene, 1,3-dihydroxy-2-methylbenzene, 3,4-methylene-dioxyphenol, 3,4-methylenedioxyaniline, 5-[(2-hydroxyethyl)amino]-1,3-benzodioxole, 6-bromo-1-hydroxy-3,4-methylenedioxybenzene, 3,4-diaminobenzoic acid, 6-hydroxybenzomorpholine, 6-amino-benzomorpholine, 3-methyl-1-phenyl-5-pyrazolone, 5,6-dihydroxyindole, 5,6-dihydroxyindoline, 5-hydroxyindole, 6-hydroxyindole, 7-hydroxy-indole, 2,3-indolinedione.

(iii) self coupling substances: 2-amino-5-methylphenole, 2-amino-6-methylphenole, 2-amino-5-ethoxyphenole or 2-propyl-amino-5-aminopyridine.

The abovementioned oxidative precursers may be present in a total amount of from about 0.01 to 12 percent of weight, in particular 0.2 to 6 percent by weight.

In general, lightening compositions according to the present invention may comprise at least one source of an oxidizing agent. Preferred oxidizing agents for use herein are water-soluble peroxygen oxidizing agents. "Water-soluble" as defined herein means that in standard condition at least 0.1 g, preferably at least 1 g, more preferably 10 g of said oxidizing agent can be dissolved in 1 liter of deionized water. The oxidizing agents are valuable for the initial solubilisation and decolourisation of the melanin (bleaching) and accelerate the oxidation of the oxidative dye precursors (oxidative dyeing) in the hair shaft.

Any oxidizing agent known in the art may be utilized in the present invention. Preferred water-soluble oxidizing agents are inorganic peroxygen materials capable of yielding hydrogen peroxide in an aqueous solution. Water-soluble peroxygen oxidizing agents are well known in the art and include hydrogen peroxide, inorganic alkali metal peroxides such as sodium periodate and sodium peroxide and organic peroxides such as urea peroxide, melamine peroxide, and inorganic perhydrate salt bleaching compounds, such as the alkali metal salts of perborates, percarbonates, perphosphates, persilicates, persulphates and the like. These inorganic perhydrate salts may be incorporated as monohydrates, tetrahydrates etc. Alkyl and aryl peroxides, and or peroxidases may also be used. Mixtures of two or more such oxidizing agents can also be used if desired. The oxidizing agents may be provided in aqueous solution or as a powder which is dissolved prior to use. Preferred for use in the compositions according to the present invention are hydrogen peroxide, percarbonate, persulphates and combinations thereof.

According to the present invention the compositions comprise from about 0.1% to about 15% by weight, preferably from about 1% to about 10% by weight, and most preferably from about 2% to about 7% by weight of an oxidizing agent.

Another preferred oxidizing agent for use herein is a source of peroxymonocarbonate ions. Preferably such a source is formed in situ from a source of hydrogen peroxide and a hydrogen carbonate ion source. Such an oxidizing agent has been found to be particularly effective at a pH of up to and including 9.5, preferably 7.5 to 9.5 more preferably about pH 9. Moreover, this system is also particularly effective in combination with a source of ammonia or ammonium ions. It has been found that this oxidizing agent can deliver improvements to the desired hair colour results particularly with regard to the delivery of high lift, whilst considerably reducing the odour, skin and scalp irritation and damage to the hair fibres.

Accordingly, any source of these ions may be utilized. Suitable sources for use herein include sodium, potassium, guanidine, arginine, lithium, calcium, magnesium, barium, ammonium salts of carbonate, carbamate and hydrocarbonate ions and mixtures thereof such as sodium carbonate, sodium hydrogen carbonate, potassium carbonate, potassium hydrogen carbonate, guanidine carbonate, guanidine hydrogen carbonate, lithium carbonate, calcium carbonate, magnesium carbonate, barium carbonate, ammonium carbonate, ammonium hydrogen carbonate and mixtures thereof. Percarbonate salts may also be utilized to provide both the source of carbonate ions and oxidizing agent. Preferred sources of carbonate ions, carbamate and hydrocarbonate ions are sodium hydrogen carbonate, potassium hydrogen carbonate, ammonium carbamate, and mixtures thereof.

According to the present invention the compositions comprise from about 0.1% to about 15% by weight, preferably from about 1 % to about 10% by weight, and most preferably from about 1 % to about 8% by weight of a hydrogencarbonate ion and from about 0.1% to about 10% by weight, preferably from about 1% to about 7% by weight, and most preferably from about 2% to about 5% by weight of a source of hydrogen peroxide.

According to the present invention the composition may further optionally comprise at least one source of alkalizing agent, preferably a source of ammonium ions and or ammonia. Any agent known in the art may be used such as alkanolamides for example monoethanolamine, diethanolamine, triethanolamine, monopropanolamine, dipropanolamine, tripropanolamine, 2-amino-2-methyl-1, 3-propanediol, 2-amino-2-methyl-1-propanol, and 2-amino-2-hydroxymethyl-1,3-propanediol and guanidium salts. Particularly, preferred alkalizing agents are those which provide a source of ammonium ions. Any source of ammonium ions is suitable for use herein. Preferred sources include ammonium chloride, ammonium sulphate, ammonium nitrate, ammonium phosphate, ammonium acetate, ammonium carbonate, ammonium hydrogen carbonate, ammonium carbamate, ammonium hydroxide, percarbonate salts, ammonia and mixtures thereof. Particularly preferred are ammonium carbonate, ammonium carbamate, ammonium hydrogen carbonate, ammonia and mixtures thereof. The compositions of the present invention may comprise from about 0.1% to about 10% by weight, preferably from about 0.5% to about 5%, most preferably from about 1% to about 3% of an alkalizing agent, preferably ammonium ions.

According to the present invention the compositions may further comprise a source of radical scavenger. As used herein the term radical scavenger refers to a species that can react with a reactive radical, preferably carbonate radicals, to convert the reactive radical by a series of fast reactions to a less reactive species.

Suitable radical scavengers for use herein include compounds according to the general formula (RSI):

$$R^{1'}\text{-}Y\text{-}C(H)(R^{3'})\text{-}R^{4'}\text{-}(C(H)(R^{5'})\text{-}Y\text{-}R^{6'})_n \qquad \text{(RSI)}$$

wherein Y is $NR^{2'}$, O, or S, preferably $NR^{2'}$, n is 0 to 2, and wherein $R^{4'}$ is monovalent or divalent and is selected from: (a) substituted or unsubstituted, straight or branched, alkyl, mono-or poly-unsaturated alkyl, heteroalkyl, aliphatic, heteroaliphatic, or heteroolefinic systems, (b) substituted or unsubstituted, mono- or poly-cyclic aliphatic, aryl, or heterocyclic systems, or (c) substituted or unsubstituted, mono-, poly-, or per-fluoro alkyl systems; the systems of (a), (b) and (c) comprising from 1 to 12 carbon atoms and 0 to 5 heteroatoms selected from O, S, N, P, and Si; and wherein $R^{4'}$ can be connected to $R^{3'}$ or $R^{5'}$ to create a 5, 6 or 7 membered ring; and wherein $R^{1'}$, $R^{2'}$, $R^{3'}$, $R^{5'}$, and $R^{6'}$ are monovalent and are selected independently from: (a), (b) and (c) described herein above, or H.

Preferably, $R^{4'}$ is selected from: (a) substituted or unsubstituted, straight or branched, alkyl, heteroalkyl, aliphatic, heteroaliphatic, or heteroolefinic systems, (b) substituted or unsubstituted, mono- or polycyclic aliphatic, aryl, or heterocyclic systems, or (c) substituted or unsubstituted, mono-, poly-, or per-fluoro alkyl systems; more preferably $R^{4'}$ is selected from (a) substituted or unsubstituted, straight or branched, alkyl, heteroalkyl, aliphatic, or heteroaliphatic systems, (b) substituted or unsubstituted, aryl, or heterocyclic systems, or (c) substituted or unsubstituted, mono-, poly-, or per-fluoro alkyl systems; more preferably substituted or unsubstituted, straight or branched, alkyl, or heteroalkyl systems.

Preferably, the $R^{4'}$ systems of (a), (b), and (c), described herein above, comprise from 1 to 8 carbon atoms, preferably from 1 to 6, more preferably from 1 to 4 carbon atoms and from 0 to 3 heteroatoms; preferably from 0 to 2 heteroatoms; most preferably from 0 to 1 heteroatoms. Where the systems contain heteroatoms, preferably they contain 1 heteroatom. Preferred heteroatoms include O, S, and N; more preferred are O, and N; and most preferred is O.

Preferably, $R^{1'}$, $R^{2'}$, $R^{3'}$, $R^{5'}$, and $R^{6'}$ are selected independently from any of the systems defined for $R^4$ above, and H.

In alternative embodiments, any of $R^{1'}$, $R^{2'}$, $R^{3'}$, $R^{4'}$, $R^{5'}$, and $R^{6'}$ groups are substituted. Preferably, the substituent(s) is selected from: (a) the group of C-linked monovalent substituents consisting of: (i) substituted or unsubstituted, straight or branched, alkyl, mono- or poly-unsaturated alkyl, heteroalkyl, aliphatic, heteroaliphatic, or heteroolefinic systems, (ii) substituted or unsubstituted, mono- or poly-cyclic aliphatic, aryl, or heterocyclic systems, or (iii) substituted or unsubstituted, mono-, poly-, or per-fluoro alkyl systems; said systems of (i), (ii) and (iii) comprising from 1 to 10 carbon atoms and 0 to 5 heteroatoms selected from O, S, N, P, and Si; (b) the group of S-linked monovalent substituents consisting of $SA^1$, SCN, $SO_2A^1$, $SO_3A^1$, $SSA^1$, $SOA^1$, $SO_2NA^1A^2$, $SNA^1A^2$, and $SONA^1A^2$; (c) the group of O-linked monovalent substituents consisting of $OA^1$, OCN and $ONA^1A^2$; (d) the group ofN-linked monovalent substituents consisting of $NA^1A^2$, $(NA^1A^2A^3)^+$, NC, $NA^1OA^2$, $NA^1SA^2$, NCO, NCS, $NO_2$, $N{=}NA^1$, $N{=}NOA^1$, $NA^1CN$, $NA^1NA^2A^3$; (e) the group of monovalent substituents consisting of $COOA^1$, $CON_3$, $CONA^1_2$, $CONA^1COA^2$, $C(=NA^1)NA^1A^2$, CHO, CHS, CN, NC, and X; and (f) the group consisting fluoroalkyl monovalent substituents consisting of mono-, poly-, or per-fluoro alkyl systems comprising from 1 to 12 carbon atoms and 0 to 4 heteroatoms.

For the groups (b) to (e), described above, $A^1$, $A^2$, and $A^3$ are monovalent and are independently selected

from: (1) H, (2) substituted or unsubstituted, straight or branched, alkyl, mono- or poly-unsaturated alkyl, heteroalkyl, aliphatic, heteroaliphatic, or heteroolefinic systems, (3) substituted or unsubstituted, mono- or poly-cyclic aliphatic, aryl, or heterocyclic systems, or (4) substituted or unsubstituted, mono-, poly-, or per-fluoro alkyl systems; said systems of (2), (3) and (4) comprising from 1 to 10 carbon atoms and 0 to 5 heteroatoms selected from O, S, N, P, and Si; and wherein X is a halogen selected from the group consisting of F, Cl, Br, and I.

Preferred substituents for use herein include those having a Hammett Sigma Para ($\sigma_p$) Value from -0.65 to +0.75, preferably from -0.4 to +0.5. Hammett Sigma Values are described in Advanced Organic Chemistry - Reactions, Mechanisms and Structure (Jerry March, 5th ed. (2001) at pages 368-375).

Alternative suitable radical scavengers for use herein are compounds according to the general formula (RSII) :

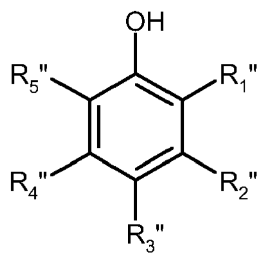

(RSII)

wherein $R_{1''}$, $R_{2''}$, $R_{3''}$, $R_{4''}$, and $R_{5''}$ are each independently selected from H, $COO^-M^+$, Cl, Br, $SO_3^-M^+$, $NO_2$, $OCH_3$, OH or a C1 to C10 primary or secondary alkyl and M is either H or alkali metal. Preferably, the above-described radical scavengers have a pKa of more than 8.5 to ensure protonation of the hydroxy goup.

Other suitable radical scavengers for use herein include those selected from group (III) benzylamine, imidazole, di-tert-butylhydroxytoluene, hydroquinone, guanine, pyrazine, piperidine, morpholine, methylmorpholine, 2methyoxyethylamine, and mixtures thereof.

Preferred radical scavengers according to the present invention are selected from the classes of alkanolamines, amino sugars, amino acids, esters of amino acids and mixtures thereof. Particularly preferred compounds are: monoethanolamine, 3-amino-1-propanol, 4-amino-1-butanol,5-amino-1-pentanol, 1-amino-2-propanol, 1-amino-2-butanol, 1-amino-2-pentanol, 1-amino-3-pentanol, 1-amino-4-pentanol, 3-amino-2-methylpropan-1-ol, 1-amino-2-methylpropan-2-ol, 3-aminopropane-1,2-diol, glucosamine, N-acetylglucosamine, glycine, arginine, lysine, proline, glutamine, histidine, sarcosine, serine, glutamic acid, tryptophan, and mixtures thereof, and the salts such as the potassium, sodium and ammonium salts thereof and mixtures thereof. Especially preferred compounds are glycine, sarcosine, lysine, serine, 2 methoxyethylamine, glucosamine, glutamic acid, morpholine, piperdine, ethylamine, 3 amino-1-propanol and mixtures thereof.

The radical scavengers according to the present invention preferably have a molecular weight of less than about 500, preferably less than about 300, more preferably less than about 250 in order to facilitate penetration of the radical scavenger into the hair fibre. The compositions of the present invention preferably comprise from about 0.1 % to about 10% by weight, preferably from about 1% to about 7% by weight of radical scavenger. The radical scavenger is also preferably selected such that it is not an identical species as the alkalizing agent. According to one embodiment of the present invention the radical scavenger may be formed insitu in the hair dyeing compositions prior to application to the hair fibres.

The colourants according to the invention produce natural looking colourations even at physiologically compatible temperatures of less than 45 ˚C. They are therefore particularly suitable for colouring human hair. For use on human hair, the colourants are usually incorporated into a hydrous cosmetic carrier. Suitable cosmetic carriers are, for example, creams, emulsions, gels or else surfactant-containing foaming solutions, such as, for example, shampoos or other preparations which are suitable for application to keratin-containing fibres. If necessary, it is also possible to incorporate the colourants into anhydrous carriers, powders, pellets or granules.

The colourant according to the invention can further comprise all additives which are customary and known for such preparations, for example perfume oils, complexing agents, waxes, preservatives, polymers, thickeners, antioxidants, alginates, guar gum, haircare substances, such as, for example, cationic polymers or lanolin derivatives, or anionic, nonionic, zwitterionic, amphoteric or cationic surface-active substances (surfactants).

Suitable polymers can include nonionic, anionic, cationic, and amphoteric or zwitterionic polymers.

Suitable anionic polymers are polymers with anionic groups or groups that can be ionized. Groups that can

be anionized are understood to be acid groups such as, for example, carboxylic acid, sulfonic acid, or phosphoric acid groups that can be deprotonated using typical bases such as, for example, organic amines or alkali- or alkaline earth hydroxides. The anionic polymers can be partially or completely neutralized with an alkaline neutralizing agent. Such types of agents in which the acidic groups are neutralized in the polymer to 50 to 100 %, or especially preferably to 70-100%, are preferred. Organic or inorganic bases can be used as the neutralizing agent. Particular examples of bases are amino alkanols such as, for example, aminomethylpropanol (AMP), triethanolamine or monoethanolamine, and also ammonia, NaOH, and KOH among others.

The anionic polymer can be a homo- or copolymer with acid group-containing monomer units derived from natural or synthetic sources, which, if necessary, can be polymerized with comonomers that contain no acid groups. Among the acid groups that can be considered are sulfonic acid, phosphoric acid, and carboxylic acid groups, of which the carboxylic acid groups are preferred. Suitable acid group-containing monomers are, for example, acrylic acid, methacrylic acid, crotonic acid, maleic acid, and maleic anhydride, maleic acid monoesters, especially the C1 to C7 alkyl monoesters of maleic acid, as well as aldehydocarboxylic acids or ketocarboxylic acids. Comonomers that are not substituted with acid groups are, for example, acrylamide, methacrylamide, alkyl- and dialkylacrylamide, alkyl and dialkylmethacrylamide, alkyl acrylate, alkyl methacrylate, vinylcaprolactone, vinylpyrrolidone, vinyl ester, vinyl alcohol, propylene glycol or ethylene glycol, amine-substituted vinyl monomers such as, for example, dialkylaminoalkyl acrylate, dialkylaminoalkyl methacrylate, monoalkylaminoalkyl acrylate, and monoalkylaminoalkyl methacrylate, wherein the alkyl groups of these monomers are preferably C1 to C7 alkyl groups, with C1 to C3 alkyl groups being especially preferred.

Suitable polymers with acid groups are especially homopolymers of acrylic acid or methacrylic acid, copolymers of acrylic acid or methacrylic acid with monomers selected from acrylic acid or methacrylic acid esters, acrylamides, methacrylamides and vinylpyrrolidone, homopolymers of crotonic acid as well as copolymers of crotonic acid with monomers selected from vinyl esters, acrylic acid or methacrylic acid esters, acrylamides and methacrylamides that are uncrosslinked or crosslinked with polyfunctional agents. A suitable natural polymer is, for example, shellac. Preferred polymers with acid groups are:

Terpolymers from acrylic acid, alkyl acrylate, and N-alkylacrylamide (INCI designation: Acrylate/Acrylamide Copolymer), especially terpolymers from acrylic acid, ethyl acrylate and N-tert-butylacrylamide; crosslinked or uncrosslinked vinyl acetate/crotonic acid copolymers (INCI designation: VA/Crotonate Copolymer); copolymers from one or more C1 to C5 alkyl acrylates, especially C2 to C4 alkyl acrylates and at least one monomer selected from acrylic acid or methacrylic acid (INCI designation: Acrylate Copolymer), e.g. terpolymers from tert-butyl acrylate, ethyl acrylate and methacrylic acid; sodium polystyrenesulfonate; vinylacetate/crotonic acid/vinyl alkanoate copolymers, for example, copolymers from vinyl acetate, crotonic acid and vinyl propionate; copolymers from vinyl acetate, crotonic acid and vinyl neodecanoate (INCI designations: VA/Crotonate/Vinyl Propionate Copolymer, VA/Crotonate/Vinyl Neodecanoate Copolymer); aminomethylpropanol acrylate copolymers; copolymers from vinylpyrrolidone and at least one further monomer selected from acrylic acid and methacrylic acid as well as, if necessary, acrylic acid esters and methacrylic acid esters; copolymers from methyl vinyl ether and maleic acid monoalkylesters (INCI designations: Ethyl Ester of PVM/MA Copolymer, Butyl Ester of PVM/MA Copolymer); aminomethylpropanol salts of copolymers from allyl methacrylate and at least one further monomer selected from acrylic acid, and methacrylic acid as well as, if necessary, acrylic acid esters and methacrylic acid esters; crosslinked copolymers from ethyl acrylate and methacrylic acid; copolymers from vinyl acetate, mono-n-butyl maleate and isobornyl acrylate; copolymers from two or more monomers selected from acrylic acid and methacrylic acid as well as, if necessary, acrylic acid esters and methacrylic acid esters; copolymers from octylacrylamide and at least one monomer selected from acrylic acid and methacrylic acid as well as, if necessary, acrylic acid esters and methacrylic acid esters (e.g. copolymer of octylacrylamide and acrylic acid and butylaminoethyl methacrylic acid); polyesters from diglycol, cyclohexanedimethanol, isophthalic acid and sulfoisophthalic acid, wherein the alkyl groups of the aforementioned polymers as a rule preferably possess 1, 2, 3, or 4 C-atoms.

Suitable zwitterionic polymers simultaneously have at least one anionic and at least one cationic charge, whereas suitable amphoteric polymers exhibit at least one acidic group (e.g. carboxylic acid or sulfonic acid group) and at least one alkaline group (e.g. amino group). Acidic groups can be deprotonated using typical bases such as, for example, organic amines or alkali- or alkaline earth hydroxides.

Preferred zwitterionic or amphoteric polymers are:

Copolymers formed from alkylacrylamide, alkylaminoalkyl methacrylate, and two or more monomers from acrylic acid and methacrylic acid as well as, if necessary, their esters, especially copolymers from octylacrylamide, acrylic acid, butylaminoethyl methacrylate, methyl methacrylate and hydroxypropyl methacrylate (INCI designation: Octylacrylamide/Acrylate/Butylaminoethyl Methacrylate Copolymer); copolymers, that are formed from at least one of a first type of monomer that possesses quaternary amino groups and at least one of a second type of monomer that possesses acid groups; copolymers from fatty alcohol acrylates, alkylamine oxide methacrylate and at least one monomer selected from acrylic acid and methacrylic acid as well as if necessary acrylic acid esters and methacrylic acid esters, especially copolymers from lauryl acrylate, stearyl acrylate, ethylamine oxide methacrylate and at least one monomer selected from acrylic acid and methacrylic acid as well as if necessary their esters; copolymers from methacryloyl ethyl

betaine and at least one monomer selected from methacrylic acid and methacrylic acid esters; copolymers from acrylic acid, methyl acrylate and methacrylamidopropyltrimethylammonium chloride (INCI designation: Polyquaternium-47); copolymers from acrylamidopropyltrimethylammonium chloride and acrylates or copolymers from acrylamide, acrylamidopropyltrimethylammonium chloride,
2-amidopropylacrylamide sulfonate, and dimethylaminopropylamine (INCI designation:
Polyquaternium-43); oligomers or polymers, producible from quaternary crotonoylbetaines or quaternary crotonoylbetaine esters.

Suitable cationic polymers are synthetic or natural polymers with cationic groups or with amine groups, particularly primary, secondary, tertiary, or quaternary amine groups; whereas natural polymers are understood to also include chemically modified polymers of natural origin.The cationic charge density will preferably be 1 to 7 meq/g. can be synthetic or natural polymers. Suitable synthetic cationic polymers are homo- or copolymers consisting of at least one of the following monomers: dialkylaminoalkyl acrylate, dialkylaminoalkyl methacrylate, monoalkylaminoalkyl acrylate, and monoalkyl aminoalkyl methacrylate, trialkyl methacryloxyalkyl ammonium, trialkyl acryloxyalkyl ammonium, dialkyl diallyl ammonium, and quaternary vinyl ammonium monomers with cyclic groups containing cationic nitrogens.

Suitable cationic polymers preferably contain quaternary amino groups. Cationic polymers can be homo- or copolymers, where the quaternary nitrogen groups are contained either in the polymer chain or preferably as substituents on one or more of the monomers. The monomers containing ammonium groups can be copolymerized with non-cationic monomers. Suitable cationic monomer are unsaturated compounds that can undergo radical polymerization, which bear at least one cationic group, especially ammonium-substituted vinyl monomers such as, for example, trialkylmethacryloxyalkylammonium, trialkylacryloxyalkylammonium, dialkyldiallyl ammonium and quaternary vinylammonium monomers with cyclic, cationic nitrogen-containing groups such as pyridinium, imidazolium or quaternary pyrrolidones, e.g. alkylvinylimidazolium, alkylvinylpyridinium, or alkylvinylpyrrolidone salts. The alkyl groups of these monomers are preferably lower alkyl groups such as, for example, C1 to C7 alkyl groups, and especially preferred are C1 to C3 alkyl groups.

The monomers containing ammonium groups can be copolymerized with non-cationic monomers. Suitable comonomers are, for example, acrylamide, methacrylamide, alkyl- and dialkylacrylamide, alkyl- and dialkylmethacrylamide, alkyl acrylate, alkyl methacrylate, vinylcaprolactone, vinylcaprolactam, vinylpyrrolidone, vinyl esters, for example vinyl acetate, vinyl alcohol, propylene glycol or ethylene glycol, wherein the alkyl groups of these monomers are preferably C1 to C7 alkyl groups, and especially preferred are C1 to C3 alkyl groups.

Suitable polymers with quaternary amino groups are, for example, those described in the CTFA Cosmetic Ingredient Dictionary under the designations Polyquaternium such as methylvinylimidazolium chloride/vinylpyrrolidone copolymer (Polyquaternium-16) or quaternized vinylpyrrolidone/dimethylaminoethyl methacrylate copolymer (Polyquaternium-11) as well as quaternary silicone polymers or silicone oligomers such as, for example, amino silicone polymers with quaternary end groups.

Preferred cationic polymers of synthetic origin are:

Poly(dimethyldiallylammonium chloride); copolymers from acrylamide and dimethyldiallyl ammonium chloride; quaternary ammonium polymers, formed by the reaction of diethyl sulfate with a copolymer from vinylpyrrolidone and dimethylaminoethyl methacrylate, especially vinylpyrrolidone/dimethylaminoethyl methacrylate methosulfate copolymer (e.g. Gafquat® 755 N, Gafquat® 734); quaternary ammonium polymers from methylvinylimidazolium chloride and vinylpyrrolidone (e.g. LUVIQUAT® HM 550); Polyquaternium-35; Polyquaternium-57; polymers from trimethylammonium ethyl methacrylate chloride; homopolymers of dimethyldiallylammonium chloride (Polyquaternium-6); copolymers from dimethyldiallylammonium chloride and acrylamide (Polyquaternium-7); copolymers from dimethyldiallylammonium chloride and acrylic acid; terpolymers from dimethyldiallylammonium chloride, sodium acrylate and acrylamide (e.g. Merquat® Plus 3300); copolymers from vinylpyrrolidone, dimethylaminopropyl methacrylamide and methacryloylaminopropyllauryldimethylammonium chloride; terpolymers from vinylpyrrolidone, dimethylaminoethyl methacrylate and vinylcaprolactam (e.g. Gaffix® VC 713); vinylpyrrolidone/methacrylamidopropyl-trimethylammonium chloride copolymers (e.g. Gafquat® HS 100); copolymers from vinylpyrrolidone and dimethylaminoethyl methacrylate; copolymers from vinylpyrrolidone, vinylcaprolactam and dimethylaminopropylacrylamide; poly- or oligoesters formed from at least one first type of monomer, that is selected from hydroxyacids substituted with at least one quaternary ammonium group; cationic amino-substituted silicones (e.g. Quaternium-80). Suitable cationic polymers that are derived from natural polymers are especially cationic derivatives of polysaccharides, for example, cationic derivatives of cellulose, starch or guar. Furthermore, chitosan and chitosan derivatives are also suitable.

Cationic polysaccharides are, for example, represented by the general formula

$$[G\text{-}O\text{-}B\text{-}NR^aR^bR^c]^+ \quad X^-$$

G is an anhydroglucose residue, for example, starch or cellulose anhydroglucose;

B is a divalent linking group, for example alkylene, oxyalkylene, polyoxyalkylene or hydroxyalkylene;
$R^a$, $R^b$, and $R^c$, independently from one another, are alkyl, aryl, alkylaryl, arylalkyl, alkoxyalkyl, or alkoxyaryl, any of which can have up to 18 C atoms, wherein the total number of C atoms in $R^a$, $R^b$, and $R^c$ is preferably a maximum of 20;
X is a conventional counter-anion, for example, a halide, acetate, phosphate, nitrate, or alkyl sulfate, preferably a chloride.
Cationic celluloses are, for example, those with the INCI names
Polyquaternium-4, Polyquaternium-10, or Polyquaternium-24. A suitable cationic guar derivative has, for example, the INCI designation Guar Hydroxypropyltrimonium Chloride.
Preferred cationic polymers derived from natural sources are:
Cationic cellulose derivatives from hydroxyethyl cellulose and diallyldimethyl ammonium chloride; cationic cellulose deviates from hydroxyethyl cellulose and trimethylammonium-substituted epoxide; chitosan and its salts; hydroxyalkyl chitosans and their salts; alkylhydroxyalkyl chitosans and their salts; N-hydroxyalkylchitosan alkyl ethers.

Suitable nonionic polymers may be synthetic or natural, whereas natural polymers are understood to also include chemically modified polymers of natural origin.
Suitable synthetic nonionic polymers are homo- or copolymers consisting of at least one of the following monomers: vinyl lactams such as, for example, vinyl pyrrolidone or vinyl caprolactam; vinyl esters such as, for example, vinyl acetate; vinyl alcohol, vinyl formamide, acrylamides, methacrylamides, alkyl acrylamides, dialkylacrylamides, alkyl methacrylamides, dialkylmethacrylamides, alkyl acrylates, alkyl methacrylates, alkyl maleimides such as, for example, ethylmaleimide or hydroxyethylmaleimide, and alkylene glycols such as, for example, propylene glycol or ethylene glycol, wherein the alkyl and/or alkylene groups of these monomers are preferably C1 to C7 alkyl groups, with C1 to C3 alkyl groups being particularly preferred.

Suitable homopolymers are, for example, those of vinylcaprolactam, vinylpyrrolidone or N-vinylformamide. Further suitable synthetic, nonionic polymers are, for example, polyacrylamides, polyethylene glycol/polypropylene glycol copolymers, copolymerides from vinylpyrrolidone and vinyl acetate, terpolymers from vinylpyrrolidone, vinyl acetate, and vinyl propionate, polyacrylamides; polyvinyl alcohols as well as polyethylene glycol/polypropylene glycol copolymers.
Suitable natural film-forming polymers are, in particular, those based on saccharide, preferably glucans, e.g. cellulose and derivatives thereof. Suitable derivatives are, in particular, those with alkyl and/or hydroxyalkyl substituents, wherein the alkyl groups can have, for example, 1 to 20, or preferably 1 to 4 C atoms, e.g. hydroxyalkyl cellulose.
Preferred nonionic polymers are:
Polyvinylpyrrolidone, polyvinylcaprolactam, vinylpyrrolidone/vinyl acetate copolymers, polyvinyl alcohol, isobutylene/ethylmaleimide/hydroxyethylmaleimide copolymer; copolymers from vinylpyrrolidone, vinyl acetate, and vinyl propionate.

Suitable surfactants can include nonionic, anionic, cationic and amphoteric or zwitterionic surfactants.
Suitable nonionic surfactants are, for example,

- ethoxylated fatty alcohols, fatty acids, fatty acid glycerides, or alkyl phenols, especially addition products of 2 to 30 mol ethylene oxide and/or 1 to 5 mol propylene oxide to C8 to C22 fatty alcohols, to C12 to C22 fatty acids, or to alkyl phenols with 8 to 15 C atoms in the alkyl group,
- C12 to C22 fatty acid mono- and diesters of addition products of 1 to 30 mol ethylene oxide to glycerol,
- addition products of 5 to 60 mol ethylene oxide to castor oil or hydrogenated castor oil,
- fatty acid sugar esters, especially esters from saccharose and one or two C8 to C22 fatty acids, INCI-name: Sucrose Cocoate, Sucrose Dilaurate, Sucrose Distearate, Sucrose Laurate, Sucrose Myristate, Sucrose Oleate, Sucrose Palmitate, Sucrose Ricinoleate, Sucrose Stearate,
- esters from sorbitan and one, two or three C8 to C22 fatty acids and a degree of ethoxylation of 4 to 20,
- polyglyceryl fatty acid esters, especially from one, two or more C8 to C22 fatty acids and polyglycerol with preferably 2 to 20 glyceryl units,
- alkylglucosides, alkyloligoglucosides, and alkylpolyglucoside with C8 to C22 alkyl groups, e.g. decyl glucoside or lauryl glucoside.

Suitable anionic surfactants are, for example, salts and esters of carboxylic acids, alkyl ether carboxylic acids, alkyl ether sulfates (e.g. lauryl ether sulfates) and alkyl sulfates (e.g. lauryl sulfates), sulfonic acids and their salts (e.g. alkylarylsufonates, alkylaryl ether sulfonates, sulfosuccinates or fatty acid isethienates), phosphoric acid esters and their salts, acylamino acids and their salts, . A comprehensive description of these anionic surfactants is found in the publication "FIEDLER - Lexikon der Hilfsstoffe" [FIEDLER - Dictionary of Adjuvants], volume 1, fifth edition (2002), pages 97 to 102, to which expressed reference is made.
Suitable amphoteric or zwitterionic surfactants are, for example, derivatives of aliphatic quaternary ammonium, phosphonium, and sulfonium compounds of the formula

$$\begin{array}{c}(R^1)_x \\ | \\ (R^2)_y\text{-}Y^{(+)}\text{-}CH_2\text{-}R^3\text{-}Z^{(-)}\end{array}$$

wherein R1 represents a straight-chain or branched-chain alkyl, alkenyl, or hydroxyalkyl group with 8 to 18 C atoms and 0 to about 10 ethylene oxide units and 0 to 1 glycerol units; Y is an N-, P-, or S-containing group; R2 is an alkyl or monohydroxyalkyl group with 1 to 3 C atoms; the total of x+y equals 2 if Y is a sulfur atom, and the total of x+y equals 3 if Y is a nitrogen atom or a phosphorus atom; R3 is an alkylene or hydroxyalkylene group with 1 to 4 C atoms, and $Z^{(-)}$ represents a carboxylate, sulfate, phosphonate, or phosphate group. Other amphoteric surfactants such as betaines are also suitable.

Examples of betaines include C8 to C18 alkylbetaines such as cocodimethylcarboxymethylbetaine, lauryldimethylcarboxymethylbetaine, lauryldimethyl-alpha-carboxyethylbetaine, cetyldimethylcarboxymethylbetaine, oleyldimethylgammacarboxypropylbetaine, and lauryl-bis-(2-hydroxypropyl)-alpha-carboxyethylbetaine; C8 to C18 sulfobetaines such as cocodimethylsulfopropylbetaine, stearyldimethylsulfopropylbetaine, lauryldimethylsulfoethylbetaine, lauryl-bis-(2-hydroxyethyl)sulfopropylbetaine; the carboxyl derivatives of imidazole, C8 to C18 alkyldimethylammonium acetate, C8 to C18 alkyldimethylcarbonylmethylammonium salts, as well as C8 to C18 fatty acid alkylamidobetaines such as, for example, coconut fatty acid amidopropylbetaine and N-coconut fatty acid amidoethyl-N-[2-(carboxymethoxy)ethyl]-glycerin (CTFA name: Cocoamphocarboxyglycinate).

Suitable cationic surfactants contain amino groups or quaternized hydrophilic ammonium groups that carry a positive charge in solution and can be represented by the general formula

$$[NR^1R^2R^3R^4]^{(+)}\ X^{(-)}$$

wherein R1 to R4, independently from one another, stand for aliphatic groups, aromatic groups, alkoxy groups, polyoxyalkylene groups, alkylamido groups, hydroxyalkyl groups, aryl groups, or alkaryl groups with 1 to 22 C atoms, wherein at least one radical has at least 6, preferably at least 8, C atoms and $X^-$ represents an anion, for example a halide, acetate, phosphate, nitrate, or alkyl sulfate, but preferably a chloride. In addition to the carbon atoms and the hydrogen atoms, the aliphatic groups can also contain cross-compounds, or other groups, such as, for example, additional amino groups.

Examples of suitable cationic surfactants are the chlorides or bromides of alkyldimethylbenzylammonium salts, alkyltrimethylammonium salts (e.g. cetyltrimethylammonium chloride or bromide), tetradecyltrimethylammonium chloride or bromide, alkyldimethylhydroxyethylammonium chlorides or bromides, dialkyldimethylammonium chlorides or bromides, alkylpyridinium salts (for example lauryl- or cetylpyridinium chloride) alkylamidoethyltrimethylammonium ether sulfates as well as compounds with cationic character such as amine oxides (e.g. alkylmethylamine oxides or alkylaminoethyldimethylamine oxides). Especially preferred are C8-22 alkyldimethylbenzylammonium compounds (e.g. lauryldimethylbenzylammonium chloride or bromide), C8-22 alkyltrimethylammonium compounds (e.g. cetyltrimethylammonium chlorid, C8-22 alkyldimethylhydroxyethyl-ammonium compounds, di-(C8-22 alkyl)-dimethylammonium compounds, C8-22 alkylpyridinium salts, C8-22 alkylamidoethyltrimethylammonium ether sulfates, C8-22 alkylmethylamine oxides, and C8-22 alkylaminoethyldimethylamine oxides.

Preferably, amphoteric or nonionic surface-active substances are used, for example betaine surfactants, propionates and glycinates, such as, for example, cocoamphoglycinates or cocoamphodiglycinates, ethoxylated surfactants with 1 to 1000 ethylene oxide units, preferably with 1 to 300 ethylene oxide units, such as, for example, glyceride alkoxylates, for example castor oil ethoxylated with 25 ethylene oxide units, polyglycolamides, ethoxylated alcohols and ethoxylated fatty alcohols (fatty alcohol alkoxylates) and ethoxylated fatty acid sugar esters, in particular ethoxylated sorbitan fatty acid esters.

The abovementioned constituents are used in the amounts customary for such purposes, for example the surface-active substances in a concentration of from 0.1 to 30 percent by weight, the care substances in an amount of from 0.1 to 5 percent by weight, and the polymers in a quantity of from 0.01 to 20 percent by weight.

The colourant according to the invention, particularly if it is a hair colourant, can be in the form of a powder or of granules, which is/are dissolved prior to application in an aqueous or aqueous-alcoholic preparation, or in the form of an aqueous or aqueous-alcoholic solution, a cream, a gel, an emulsion or an aerosol foam. The hair colourant according to the invention can be either in the form of a single-component preparation or in the form of a multicomponent preparation, for example in the form of a two-component preparation, where the direct dyes of the general formula (I) or (II) and other

dyes (e.g. oxidative dye precursors) are packaged separately from the other ingredients (e.g. hydrogen peroxide) and the ready-to-use hair colourant is prepared immediately prior to application by mixing the two components.

The colourant according to the invention generally has a pH of from about 2 to 11, preferably about 3 to 10, in particular a neutral to basic pH from about 7 to 10 in case of direct dyes according to the general formula (I) and an acid pH from about 3 to 4 in case of direct dyes according to the general formula (II).

Both, organic and also inorganic acids are suitable for adjusting the pH according to the invention. Examples of suitable acids are the following acids: $\alpha$-hydroxycarboxylic acids, such as, for example, glycolic acid, lactic acid, tartaric acid, citric acid or malic acid, ascorbic acid, gluconolactone, acetic acid, hydrochloric acid or phosphoric acid, and mixtures of these acids. Suitable alkalising media are in particular sodium carbonate, sodium bicarbonate, alkanoleamines such as monoethanoleamine or triethanoleamine, ammonia, aminopropylpropanole and sodium hydroxide and mixtures of these bases.

The colourant according to the invention, depending on its purpose, may be applied in the presence or absence of oxidizing agents such as hydrogen peroxide.

The colourant according to the invention is generally used by applying to the hair an appropriate amount of the hair colourant, normally about 30 to 120 grams depending on the length of hair, leaving the hair colourant to act at about 15 to 45 ˚C for about 1 to 60 minutes, preferably 5 to 30 minutes, then thoroughly rinsing the hair with water, optionally washing with a shampoo and/or after-treating with a hair-conditioning composition and finally drying.

Where appropriate, the colourant is mixed with an oxidizing agent prior to application.

In addition, if no oxidizing agents are added to the colouring mass, the colourant described above can also comprise natural or synthetic polymers or modified polymers of natural origin customary for cosmetic compositions, through which setting of the hair is achieved at the same time as the colouring. Such compositions are generally referred to as tinting setting compositions or colour setting compositions.

Of the synthetic polymers known for this purpose in cosmetics, mention may be made, for example, of polyvinylpyrrolidone, polyvinyl acetate, polyvinyl alcohol or polyacrylic compounds, such as polyacrylic acid or polymethacrylic acid, basic polymers of esters of polyacrylic acid, polymethacrylic acid and aminoalcohols, for example their salts or quaternization products, polyacrylonitrile, polyvinyl acetates and copolymers of such compounds, such as, for example, polyvinylpyrrolidone vinyl acetate; whereas natural polymers or modified natural polymers which may be used are, for example, chitosan (deacetylated chitin) or chitosan derivatives. The abovementioned polymers may be present in these tinting setting compositions or colour setting compositions in the amounts customary for such compositions, in particular in an amount of from about 1 to 5 percent by weight. The pH of the colour styling foam/mousse according to the inverntion, is particularly of from about 6 to 9.

The hair colourant with additional setting is used in a known and customary manner by wetting the hair with the setting composition, fixing (arranging) the hair in the hairstyle and then drying.

The colourant according to the invention permits an even, intense and long-lasting colouration of keratin fibres (for example human hair, wool or furs) without noteworthy discolouration of the skin and/or scalp.

The examples below are intended to illustrate the subject-matter of the invention in more detail without limiting it thereto.

**Examples**

**Example 1:** Synthesis of 4-{2-{(*E*)-[4-(diethylamino)phenyl]diazenyl}-5-[(1,3-dioxo-1,3-dihydro-2*H*-inden-2-ylidene)methyl]-1,3-thiazol-4-yl}-1,1-dimethylpiperazin-1-ium methylsulfate

Step 1: Synthesis of 2-{[2-{(*E*)-[4-(diethylamino)phenyl]diazenyl}-4-(4-methyl-1-piperazinyl)-1,3-thiazol-5-yl]methylene}-1*H*-indene-1,3(2*H*)-dione

1.00 g (2.20 mmol) 2-[(4-chloro-2-{(*E*)-[4-(diethylamino)phenyl]diazenyl}-1,3-thiazol-5-yl)methylene]-1*H*-indene-1,3(2*H*)-dione and 0.30 g (2.70 mmol) 1-methylpiperazine were dissolved in 40 ml tetrahydrofurane (THF) and heated under stirring for 5 hours when thin layer chromatography (TLC) analysis indicated complete consumption of the starting material. After cooling to room temperature the formed precipitate was filtered off, washed with THF and dried in vacuum at 40 ˚C to obtain a dark green powder.

Yield: 0.95 g (84%)

$^1$H NMR ($d_6$-DMSO/300 MHz): $\delta$ =1.20 (t, J= 6.9 Hz, 6H, 2x$CH_3$), 2.34 (s, 3H, $CH_3$), 3.01-3.04 (m, 4H, 2x$CH_2$), 3.57-3.62 (m, 4H, 2x$CH_2$), 3.84-3.86 (m, 4H, 2x$CH_2$), 6.96 (d, J= 9.3 Hz, 2H, phenyl), 7.75 (s, 1H), 7.77 (s, 4H), 7.85 (d, J= 9.0 Hz, 2H, phenyl).

Step 2: Synthesis of 4-{2-{(*E*)-[4-(diethylamino)phenyl]diazenyl}-5-[(1,3-dioxo-1,3-dihydro-2*H*-inden-2-ylidene)methyl]-1,3-thiazol-4-yl}-1,1-dimethylpiperazin-1-ium methylsulfate

0.93 g (1.80 mmol) 2-{[2-{(*E*)-[4-(diethylamino)phenyl]diazenyl}-4-(4-methyl-1-piperazinyl)-1,3-thiazol-5-yl] methylene}-1*H*-indene-1,3(2*H*)-dione was dissolved in 20 ml acetonitrile. After addition of 0.5 g (3.70 mmol) dimethylsulfate, the mixture was heated under reflux for 24 hours. After cooling to room temperature the formed precipitate was filtered off and dried in vacuum at 40 ˚C to obtain a dark green solid.
Yield: 0.85 g (74%)
$^1$H NMR ($d_6$-DMSO/300 MHz): δ = 1.22 (t, J= 6.6 Hz, 6H, 2x$CH_3$), 2.91 (s, 3H, $CH_3$, methylsulfate), 3.29 (s, 3H, $CH_3$), 3.35 (s, 8H, 4x$CH_2$), 3.38 (s, 3H, $CH_3$), 3.62-3.64 (m, 4H, 2x$CH_2$), 7.00 (d, J= 9.3 Hz, 2H, phenyl), 7.75 (s, 1H), 7.84 (s, 4H), 7.88 (d, J= 9.0 Hz, 2H, phenyl).

**Example 2:** Synthesis of2-[{4-[(4-chloro-5-formyl-1,3-thiazol-2-yl)diazenyl]phenyl}(ethyl)amino]ethyl hydrogen sulfate

Step 1: Synthesis of 4-chloro-2-((*E*)-{4-[ethyl(2-hydroxyethyl)amino[phenyl}-diazenyl)-1,3-thiazole-5-carbaldehyde

1.3 g (18.56 mmol) sodium nitrite was added in small portions to 13.2 g conc. sulphuric acid and cooled to 0 ˚C and diluted with 4.8 ml propionic acid and 19.2 ml acetic acid. 3.0 g (18.45 mmol) 2-amino-4-chloro-1,3-thiazole-5-carbaldehyde were then added in small portions. The viscous reaction mixture was stirred for 2 hours at 0 ˚C. This mixture was then added slowly to a solution of 3.2 g (18.56 mmol) 2-[ethyl(phenyl)amino]ethanol in 180 ml water, containing 6 ml sulphuric acid. After stirring for another 2 hours at room temperature, the obtained precipitate was filtered off, washed with water and dried at 40 ˚C in vacuum.
Yield: 3.58 g (57%)
$^1$H NMR ($d_6$-DMSO/300 MHz): δ = 1.20 (t, J= 6.9 Hz, 3H, $CH_3$), 3.47-3.55 (m, 6H, 3x$CH_2$), 3.96 (s, br., 1H, OH), 7.03 (d, J= 9.0Hz, 2H, phenyl), 7.84 (d, J= 9.0 Hz, 2H, phenyl), 9.92 (s, 1H, formyl).

Step 2: Synthesis of 2-{4-[(4-chloro-5-formyl-1,3-thiazol-2-yl)diazenyl]phenyl}(ethyl)aminolethyl hydrogen sulfate

0.8 g (2.36 mmol) 4-chloro-2-((*E*)-{4-[ethyl(2-hydroxyethyl)amino]phenyl}-diazenyl)-1,3-thiazole-5-carbaldehyde were dissolved in 35 ml THF and cooled to 0 ˚C. Then 0.28 g (2.36 mmol) chlorosulfonic acid were added slowly drop by drop with stirring. After the addition was completed, the reaction mixture was heated under reflux for 4 hours, allowed to cool to room temperature and stirred overnight. After further cooling in an ice bath the formed precipitate was filtered off, washed with a little cold THF and dried at 40 ˚C in vacuum.
Yield: 1.05 g (100%, containing traces of crystal water)
$^1$H NMR ($d_6$-DMSO/ 300 MHz): δ = 1.20 (t, J= 6.9 Hz, 3H, $CH_3$), 3.66-3.68 (m, 2H, $CH_2$), 3.80-3.82 (m, 2H, $CH_2$), 3.95-3.97 (m, 2H, $CH_2$), 4.77 (s, br., OH, +crystal water), 7.06 (d, J= 9.0 Hz, 2H, phenyl), 7.85 (d, J= 9.3 Hz, 2H, phenyl), 9.93 (s, 1H, formyl).

**Example 3:** Synthesis of 2-[{4-[(*E*)-{4-chloro-5-[(1,3-dioxo-1,3-dihydro-2*H*-inden-2-ylidene)methyl]-1,3-thiazo 1-2-yl}diazenyl]phenyl}(ethyl)amino]ethyl hydrogen sulfate

Step 1: Synthesis of 2-{[4-chloro-2-((*E*)-14-[ethyl(2-hydroxyethyl)aminol-12henyl}diazenyl)-1,3-thiazol-5-yl]methylidene}-1*H*-indene-1,3(2*H*)-dione

3.5 g (10.33 mmol) 4-chloro-2-((*E*)-{4-[ethyl(2-hydroxyethyl)amino]phenyl}-diazenyl)-1,3-thiazole-5-carbaldehyde and 2.33 g (15.50 mmol) 1*H*-indene-1,3(2*H*)-dione were dissolved in 90 ml ethanole and stirred under reflux. After 6 hours the mixture was allowed to cool to room temperature. The formed precipitate was filtered, washed with ethanol and dried in vacuum at 40 ˚C to obtain a dark powder.
Yield: 3.93 g (82%)
$^1$H NMR ($d_6$-DMSO/300 MHz): δ = 1.22 (t, J= 7.2 Hz, 3H, $CH_3$), 3.65-3.67 (m, 6H, 3x$CH_2$), 7.02 (d, J= 9.0 Hz, 2H, phenyl), 7.78 (s, 1H), 7.84 (d, J= 9.3 Hz, 2H, phenyl), 7.93 (s, 4H).

Step 2: Synthesis of 2-[{4-[(*E*)-{4-chloro-5-[(1,3-dioxo-1,3-dihydro-2*H*-inden-2-ylidene)methyl]-1,3-thiazol-2-yl}diazenyl]phenyl}(ethyl)amino]ethyl hydrogen sulfate

0.5 g (1.07 mmol) 2-{[4-chloro-2-((*E*)-{4-[ethyl(2-hydroxyethyl)amino]-phenyl}-diazenyl)-1,3-thiazol-5-yl]methylidene}-1*H*-indene-1,3(2*H*)-dione were dissolved in 50 ml THF and cooled to 0 ˚C. Then 0.19 g (1.60 mmol) chlorosulfonic acid were added slowly drop by drop with stirring. After the addition was completed, the reaction mixture was heated

under reflux for 4 hours, allowed to cool to room temperature and stirred overnight. After further cooling in an ice bath the formed precipitate was filtered off, washed with a little cold THF and dried at 40 ˚C in vacuum.
Yield: 0.53 g (90%)
$^1$H NMR ($d_6$-DMSO/ 300 MHz): δ = 1.23 (t, J= 6.6 Hz, 3H, $CH_3$), 3.43-3.98 (m, 6H, $3xCH_2$), 5.65 (s, br., OH, +crystal water), 7.00 (d, J= 8.7 Hz, 2H, phenyl), 7.75 (s, 1H), 7.82 (d, J= 9.0 Hz, 2H, phenyl), 7.98 (s, 4H).

**Example 4:** Synthesis of 2-[{4-[(*E*)-{5-[(1,3-dioxo-1,3-dihydro-2*H*-inden-2-ylidene)methyl]-4-(1-piperidinyl)-1,3-thiazol-2-yl} diazenyl]phenyl} (ethyl)amino]ethyl hydrogen sulfate

Step 1: Synthesis of 2-{[2-((*E*)-{4-[ethyl(2-hydroxyethyl)aminol]phenyl}-diazenyl)-4-(1-piperidinyl)-1,3-thiazol-5-yl] methylene}-1*H*-indene-1,3(2*H*)-dione

0.5 g (1.07 mmol) 2-{[4-chloro-2-((*E*)-{4-[ethyl(2-hydroxyethyl)amino]phenyl}-diazenyl)-1,3-thiazol-5-yl]methylene}-1*H*-indene-1,3(2*H*)-dione were dissolved in 20 ml THF. Then 0.2 g (2.14 mmol) piperidine were added dropwise and the mixture was stirred under reflux for 6 hours. After cooling to room temperature, stirring was continued overnight. The formed piperidine hydrochloric salt was separated by filtration and the solvent removed with a rotary evaporator. The resulting precipitate containing the crude product was recrystallized with toluene to obtain a dark powder.
Yield: 0.36 g (66%)
$^1$H NMR ($d_6$-DMSO/ 300 MHz): δ = 1.21 (t, J= 6.6 Hz, 3H, $CH_3$), 1.65-1.74 (m, 6H, $3xCH_2$), 3.64-3.66 (m, 6H, $3xCH_2$), 3.83-3.85 (m, 4H, $2xCH_2$), 4.96 (s, 1H, OH), 7.02 (d, J= 9.3 Hz, 2H, phenyl), 7.75 (s, 1H), 7.76 (s, 4H), 7.87 (d, J= 9.0 Hz, 2H, phenyl).

Step 2: Synthesis of 2-[{4-[(*E*)-{5-[(1,3-dioxo-1,3-dihydro-2*H*-inden-2-ylidene)methyl]-4-(1-piperidinyl)-1,3-thiazol-2-yl}-diazenyl]phenyl}(ethyl)amino]ethyl hydrogen sulfate

0.32 g (0.62 mmol) 2-{[2-((*E*)-{4-[ethyl(2-hydroxyethyl)amino]phenyl}-diazenyl)-4-(1-piperidinyl)-1,3-thiazol-5-yl]methylene}-1*H*-indene-1,3(2*H*)-dione were dissolved in 33 ml THF and cooled to 0 ˚C. Then 0.11 g (0.93 mmol) chlorosulfonic acid were added slowly drop by drop with stirring. After the addition was completed, the reaction mixture was heated under reflux for 4 hours, allowed to cool to room temperature and stirred overnight. After further cooling in an ice bath the formed precipitate was filtered off, washed with a little cold THF and dried at 40 ˚C in vacuum.
Yield: 0.10 g (27%)
$^1$H NMR ($d_6$-DMSO/ 300 MHz): δ = 1.22 (t, J= 6.6 Hz, 3H, $CH_3$), 1.70-1.75 (m, 6H, $3xCH_2$), 3.67-3.69 (m, 2H, $CH_2$), 3.82-3.86 (m, 6H, $3xCH_2$), 3.95-3.97 (m, 2H, $CH_2$), 5.57 (s, br., OH, +crystal water), 7.05 (d, J= 9.0 Hz, 2H, phenyl), 7.77 (s, 1H), 7.78 (s, 4H), 7.87 (d, J= 9.0 Hz, 2H, phenyl).

**Example 5:** Hair Colourant

| | |
|---|---|
| 2.5 mmol | dye according to formula (I) |
| 5.0 g | ethanol |
| 4.0 g | decylpolyglucose |
| 0.2 g | ethylenediaminotetraacetic acid disodium salt hydrate |
| ad 100.0 g | water, demineralized |

**Examples 6 to 8:**

| | |
|---|---|
| 2.5 mmol | dye according to formula (II) |
| 0.2 g | 1-hydroxyethane-1,1-diphosphone acid tetrasodium salt |
| 1.3 g | citric acid |
| 25.0 g | ethanol |
| 10.0 g | 1,2-propanediol |
| 9.0 g | benzylalcohol |
| 0.5 g | vinylpyrrolidone/vinylacetate copolymer |
| 0.4 g | acrylic acid polymer crosslinked with a polyfunctional agent |
| ad 100.0 g | water, demineralized |

The hair colouring for examples 5 to 8 is carried out by applying an amount of the colourant sufficient for the

hair colouring to the hair and distributing it evenly using a brush. After a contact time of 30 minutes at 40 °C, the hair is rinsed with lukewarm water, washed with a shampoo, rinsed with lukewarm water and then dried.

In addition the dye of example 5 was also treated in the presence of hydrogen peroxide as an oxidizing agent at a basic and a neutral pH. For the second treatment 5 g of the above colour carrier mass according to example 5 were mixed with 5 g of a 6% strength hydrogen peroxide solution. For the third treatment, the pH was adjusted to a basic pH of 9 by using 25% strength ammonia. The colouring results are summarized in table 1 below.

Table 1:

| **Example No**. | **Compound of the formulas (I)/(II) as in example 5-8** | **Colour shade after colouring** | **Colour measurement values after colouring** |
|---|---|---|---|
| **5** | 4-{2-{(*E*)-[4-(diethylamino)-phenyl]diazenyl}-5-[(1,3-dioxo-1,3-dihydro-2*H*-inden-2-ylidene)methyl]-1,3-thiazol-4-yl}-1,1-dimethylpiperazin-1-ium methylsulfate | iron grey (without hydrogen peroxide)<br><br>iron grey (with hydrogen peroxide, pH=7)<br><br>pale grey (with hydrogen peroxide, pH= 9) | L: 34.37<br>a: - 0.10<br>b: - 9.04<br><br>L: 38.23<br>a: + 0.43<br>b: - 9.99<br><br>L: 50.89<br>a: + 4.83<br>b: + 2.49 |
| **6** | 2-[{4-[(*E*)-(4-chloro-5-formyl-1,3-thiazol-2-yl)diazenyl]phenyl}(ethyl)-amino]ethyl hydrogen sulfate | intense dark blue | L: 16.71<br>a: + 7.41<br>b: - 10.10 |
| 7 | 2-[{4-[(*E*)-{4-chloro-5-[(1,3-dioxo-1,3-dihydro-2*H*-inden-2-ylidene)methyl]-1,3-thiazol-2-yl} diazenyl]phenyl}(ethyl)-amino]ethyl hydrogen sulfate | steel blue | L: 30.07<br>a: +4.41<br>b: - 23.16 |
| **8** | 2-[{4-[(*E*)-{5-[(1,3-dioxo-1,3-dihydro-2*H*-inden-2-ylidene)methyl]-4-(1-piperidinyl)-1,3-thiazol-2-yl} diazenyl]phenyl}(ethyl)-amino]ethyl hydrogen sulfate | black | L: 18.50<br>a: -0.03<br>b: -0.78 |

Unless otherwise indicated, all percentages in the present patent application are by weight.

The dimensions and values disclosed herein are not to be understood as being strictly limited to the exact numerical values recited. Instead, unless otherwise specified, each such dimension is intended to mean both the recited value and a functionally equivalent range surrounding that value. For example, a dimension disclosed as "40 mm" is intended to mean "about 40 mm".

## Claims

1. An agent for the non-oxidative colouring of keratin fibres, **characterized in that** it comprises at least one dye according to formula (I) or (II),

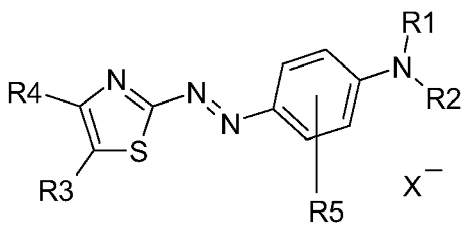

(I)

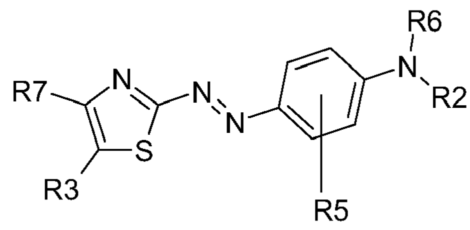

(II)

in which **R1** and **R2** may be identical or different and, independently of one another, are hydrogen, a benzyl group, a $C_1$-$C_6$-alkyl group, a $C_2$-$C_4$-hydroxyalkyl group, a $C_2$-$C_4$-cyanoalkyl group or a $C_4$-$C_6$-polyhydroxyalkyl group, wherein the alkyl groups can be both branched and linear; and
**R3** is a group according to the general formulas (III), (IV), (V), (VI) or (VII),

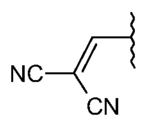

(III)

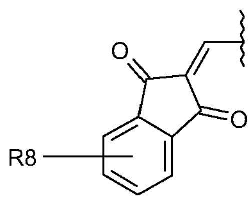

(IV)

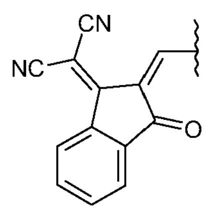

(V)

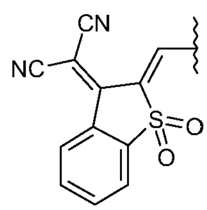

(VI)

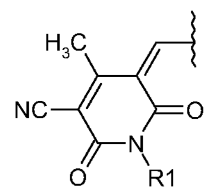

(VII)

or a formyl group, in which **R8** is a hydrogen, a halogen atom, a $C_1$-$C_6$-alkyl group, or a nitro group and in which **R1** has the abovementioned meaning; and
**R4** is a cationic group according to the general formula (VIII) or (IX),

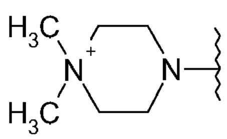

(VIII)

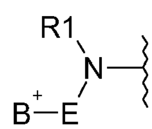

(IX)

in which **E** may be a $C_1$-$C_4$-alkyl group, a $C_3$-$C_6$-cycloalkyl group or an arylgroup; and **R1** has the above-mentioned meaning, and **B**$^+$ may be

a) a cationic, aromatic heterocyclic ammonium compound, preferably a cationic derivative of N-methyl-imidazole, N-allyl-imidazole, 2-ethyl-imidazole or 1,2-dimethyl-imidazole or a cationic derivative of pyridine, 4-dimethylamino-pyridine, pyrimidine, pyrazole, N- methylpyrazole or chinoline; or
b) a non-aromatic heterocyclic ammonium compound, in particular a cationic derivative of N-methyl-morpholine, N-ethyl-morpholine or 1-methyl-piperidine; or
c) a cationic alkylammonium compound or an arylammonium compound according to the formula $NR_aR_bR_c$, in which $\mathbf{R_a}$, $\mathbf{R_b}$ and $\mathbf{R_c}$, independently of each other, are a benzyl rest, a phenyl rest or a $C_1$-$C_6$-alkyl rest, preferably a methyl group, an ethyl group, a propyl group, an isopropyl group or a butyl group, whereas the prementioned alkyl groups may be unsubstituted or substituted with one or more hydroxy groups or amino groups; or
d) a cationic phosphonium group, e.g. a tributyl phosphonium group, preferably a trimethyl phosphonium group or a triethyl phosphonium group; and

**R5** is hydrogen, a halogen atom, a $C_1$-$C_6$-alkyl group, a hydroxy group, a $C_1$-$C_6$-alkyloxy group, an amino group or an amino-acetyl group; and
R6 is a $C_1$-$C_6$-alkylsulfonic acid group, a $C_1$-$C_6$-alkylsulfate group (a $C_1$-$C_6$-alkylsulfonic acid ester) or their corresponding sodium or potassium salts, respectively; and
**R7** is a chloro atom, a pyrrolidine rest, a piperidine rest, or a *N*-methyl-piperazine rest or an aliphatic secondary amine rest bearing two $C_1$-$C_4$-alkyl chains which may be identical or different; and
**X**$^-$ is a anionic counterion.

2. An agent for the oxidative colouring of keratin fibres, **characterized in that** it comprises at least one oxidizing agent, at least one oxidative dye precursor and at least one dye according to formula (I) or (II), and comprises at least one dye according to formula (I) or (II),

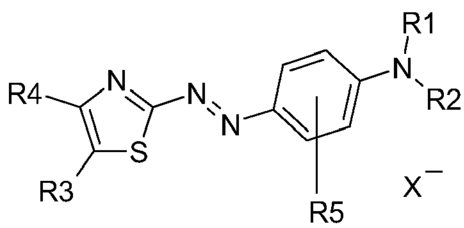
(I)

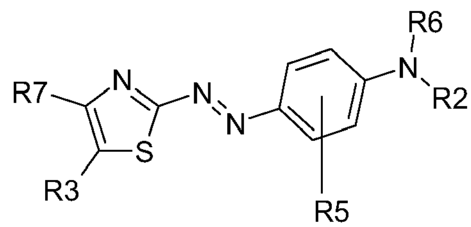
(II)

in which **R1** and **R2** may be identical or different and, independently of one another, are hydrogen, a benzyl group, a $C_1$-$C_6$-alkyl group, a $C_2$-$C_4$-hydroxyalkyl group, a $C_2$-$C_4$-cyanoalkyl group or a $C_4$-$C_6$-polyhydroxyalkyl group, wherein the alkyl groups can be both branched and linear; and
**R3** is a group according to the general formulas (III), (IV), (V), (VI) or (VII),

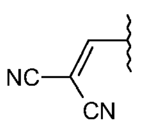
(III)

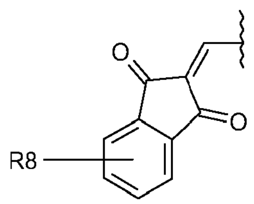
(IV)

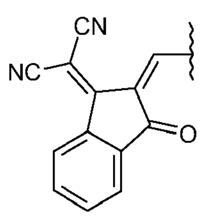
(V)

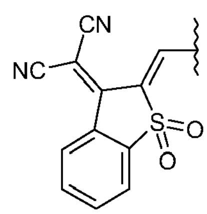
(VI)

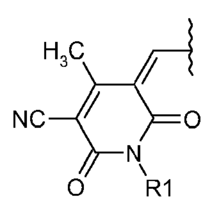
(VII)

or a formyl group, in which **R8** is a hydrogen, a halogen atom, a $C_1$-$C_6$-alkyl group, or a nitro group and in which **R1** has the abovementioned meaning; and
**R4** is a cationic group according to the general formula (VIII) or (IX),

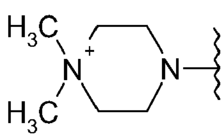
(VIII)

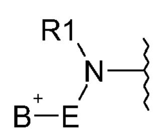
(IX)

in which E may be a $C_1$-$C_4$-alkyl group, a $C_3$-$C_6$-cycloalkyl group or an arylgroup; and **R1** has the above-mentioned meaning, and **B$^+$** may be

a) a cationic, aromatic heterocyclic ammonium compound, preferably a cationic derivative of N-methyl-imidazole, N-allyl-imidazole, 2-ethyl-imidazole or 1,2-dimethyl-imidazole or a cationic derivative of pyridine, 4-dimethylamino-pyridine, pyrimidine, pyrazole, N- methylpyrazole or chinoline; or

b) a non-aromatic heterocyclic ammonium compound, in particular a cationic derivative of N-methyl-morpholine, N-ethyl-morpholine or 1-methyl-piperidine; or

c) a cationic alkylammonium compound or an arylammonium compound according to the formula $NR_aR_bR_c$, in which $\mathbf{R_a}$, $\mathbf{R_b}$ and $\mathbf{R_c}$, independently of each other, are a benzyl rest, a phenyl rest or a $C_1$-$C_6$-alkyl rest, preferably a methyl group, an ethyl group, a propyl group, an isopropyl group or a butyl group, whereas the prementioned alkyl groups may be unsubstituted or substituted with one or more hydroxy groups or amino groups; or

d) a cationic phosphonium group, e.g. a tributyl phosphonium group, preferably a trimethyl phosphonium group or a triethyl phosphonium group; and

**R5** is hydrogen, a halogen atom, a $C_1$-$C_6$-alkyl group, a hydroxy group, a $C_1$-$C_6$-alkyloxy group, an amino group or an amino-acetyl group; and
**R6** is a $C_1$-$C_6$-alkylsulfonic acid group, a $C_1$-$C_6$-alkylsulfate group (a $C_1$-$C_6$-alkylsulfonic acid ester) or their corresponding sodium or potassium salts, respectively; and
**R7** is a chloro atom, a pyrrolidine rest, a piperidine rest, or a *N*-methyl-piperazine rest or an aliphatic secondary amine rest bearing two $C_1$-$C_4$-alkyl chains which may be identical or different; and
$X^-$ is a anionic counterion.

**3.** An agent for the lightening of keratin fibres, **characterized in that** it comprises at least one oxidizing agent and at least one dye according to formula (I) or (II),

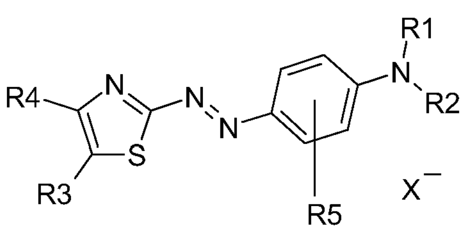

(I)

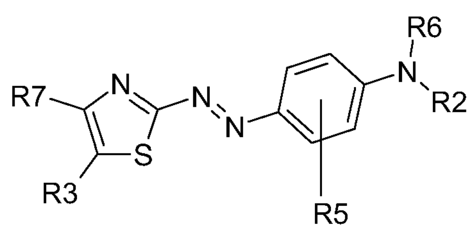

(II)

in which **R1** and **R2** may be identical or different and, independently of one another, are hydrogen, a benzyl group, a $C_1$-$C_6$-alkyl group, a $C_2$-$C_4$-hydroxyalkyl group, a $C_2$-$C_4$-cyanoalkyl group or a $C_4$-$C_6$-polyhydroxyalkyl group, wherein the alkyl groups can be both branched and linear; and
**R3** is a group according to the general formulas (III), (IV), (V), (VI) or (VII),

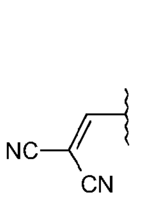

(III)

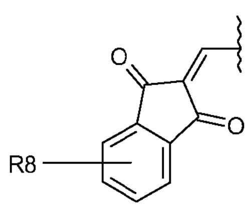

(IV)

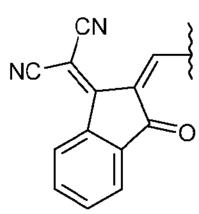

(V)

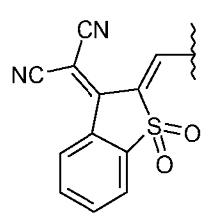

(VI)

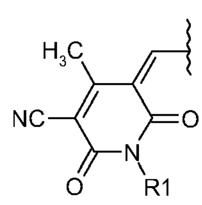

(VII)

or a formyl group, in which **R8** is a hydrogen, a halogen atom, a $C_1$-$C_6$-alkyl group, or a nitro group and in which **R1** has the abovementioned meaning; and
**R4** is a cationic group according to the general formula (VIII) or (IX),

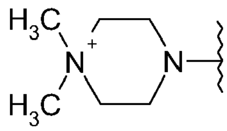

(VIII)

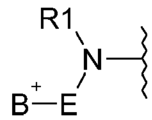

(IX)

in which **E** may be a $C_1$-$C_4$-alkyl group, a $C_3$-$C_6$-cycloalkyl group or an arylgroup; and **R1** has the above-mentioned meaning, and $\mathbf{B}^+$ may be

a) a cationic, aromatic heterocyclic ammonium compound, preferably a cationic derivative of N-methyl-imidazole, N-allyl-imidazole, 2-ethyl-imidazole or 1,2-dimethyl-imidazole or a cationic derivative of pyridine, 4-dimethylamino-pyridine, pyrimidine, pyrazole, N- methylpyrazole or chinoline; or
b) a non-aromatic heterocyclic ammonium compound, in particular a cationic derivative of N-methyl-morpholine, N-ethyl-morpholine or 1-methyl-piperidine; or
c) a cationic alkylammonium compound or an arylammonium compound according to the formula $NR_aR_bR_c$, in which $\mathbf{R_a}$, $\mathbf{R_b}$ and $\mathbf{R_c}$, independently of each other, are a benzyl rest, a phenyl rest or a $C_1$-$C_6$-alkyl rest, preferably a methyl group, an ethyl group, a propyl group, an isopropyl group or a butyl group, whereas the prementioned alkyl groups may be unsubstituted or substituted with one or more hydroxy groups or amino groups; or
d) a cationic phosphonium group, e.g. a tributyl phosphonium group, preferably a trimethyl phosphonium group or a triethyl phosphonium group; and

**R5** is hydrogen, a halogen atom, a $C_1$-$C_6$-alkyl group, a hydroxy group, a $C_1$-$C_6$-alkyloxy group, an amino group or an amino-acetyl group; and
**R6** is a $C_1$-$C_6$-alkylsulfonic acid group, a $C_1$-$C_6$-alkylsulfate group (a $C_1$-$C_6$-alkylsulfonic acid ester) or their corresponding sodium or potassium salts, respectively; and
**R7** is a chloro atom, a pyrrolidine rest, a piperidine rest, or a *N*-methyl-piperazine rest or an aliphatic secondary amine rest bearing two $C_1$-$C_4$-alkyl chains which may be identical or different; and
$X^-$ is a anionic counterion.

**4.** The agent as claimed in claim 1, **characterized in that** it comprises at least one polymer customary for cosmetic agents, selected from the group consisting of natural polymers, synthetic polymers and modified polymers of natural origin, and is in the form of a tinting setting composition or colour setting composition.

**5.** The agent as claimed in claim 2, **characterized in that** the oxidizing agent is is selected from the group consisting of hydrogen peroxide and its addition compounds onto urea, melamine, sodium borate and sodium carbonate.

**6.** The agent as claimed in claim 3, **characterized in that** the oxidizing agent is is selected from the group consisting of hydrogen peroxide and its addition compounds onto urea, melamine, sodium borate and sodium carbonate, and persulfates.

**7.** The agent as claimed in one of claims 1 to 6, **characterized in that** the counterion $X^-$ is selected from the group consisting of sulphate anions, methylsulphate anions, phosphate anions, hydrogenphosphate anions, oxalate anions, formate anions, acetate anions, citrate anions, tartrate anions, malonate anions, pyruvate anions and halogen anions.

**8.** The agent as claimed in one of claims 1 to 7, **characterized in that** the dye according to formula (I) and (II) is selected from the group consisting of 4-{2-{(*E*)-[4-(dimethylamino)phenyl]diazenyl}-5-[(1,3-dioxo-1,3-dihydro-2*H*-inden-2-ylidene)methyl]-1,3-thiazol-4-yl}-1,1-dimethylpiperazin-1-ium methylsulfate, 4-{2-{(*E*)-[4-(diethylamino)-phenyl]diazenyl}-5-[(1,3-dioxo-1,3-dihydro-2*H*-inden-2-ylidene)methyl]-1,3-thiazol-4-yl}-1,1-dimethylpiperazin-1-ium methylsulfate, 4-(5-(2,2-dicyanovinyl)-2- {(*E*)-[4-(dimethylamino)phenyl]diazenyl}-1,3-thiazol-4-yl)-1,1-dimethylpiperazin-1-ium methylsulfate, 4-(5 -(2,2-dicyanovinyl)-2- {(*E*)-[4-(diethylamino)phenyl]diazenyl} -1,3-thiazol-4-yl)-1,1-dimethylpiperazin-1-ium methylsulfate, 4-(5- {(*Z*)-[1-(dicyanomethylene)-3-oxo-1,3-dihydro-2*H*-inden-2-ylidene]methyl} -2- {(*E*)-[4-(dimethylamino)phenyl]diazenyl}-1,3-thiazol-4-yl)-1,1-dimethylpiperazin-1-ium methylsulfate, 4-(5- {(*Z*)-[1-(dicyanomethylene)-3-oxo-1,3-dihydro-2*H*-inden-2-ylidene]methyl} -2- {(*E*)-[4-(diethylamino)phenyl]diazenyl}-1,3-thiazol-4-yl)-1,1-dimethylpiperazin-1-ium methylsulfate, 2-[{4-[(*E*)-{5-[(1,3-dioxo-1,3-dihydro-2*H*-inden-2-ylidene)methyl]-4-(1-piperidinyl)-1,3-thiazol-2-yl}diazenyl]phenyl}(ethyl)amino]ethyl hydrogen sulfate, sodium 2-[{4-[(*E*)-{5-[(1,3-dioxo-1,3-dihydro-2*H*-inden-2-ylidene)methyl]-4-(1-piperidinyl)-1,3-thiazol-2-yl} diazenyl] phenyl}(ethyl)amino]ethyl sulfate, 2-[{4-[(*E*)-{5-[(1,3-dioxo-1,3-dihydro-2*H*-inden-2-ylidene)methyl]-4-(1-pyrrolidinyl)-1,3-thiazol-2-yl}diazenyl]phenyl}(ethyl)amino]ethyl hydrogen sulfate, sodium 2-[{4-[(*E*)-{5-[(1,3-dioxo-1,3-dihydro-2*H*-inden-2-ylidene)methyl]-4-(1-pyrrolidinyl)-1,3-thiazol-2-yl}diazenyl]phenyl}(ethyl)amino]ethyl sulfate, 2-[{4-[(*E*)-{4-chloro-5-[(1,3-dioxo-1,3-dihydro-2*H*-inden-2-ylidene)methyl]-1,3-thiazol-2-yl}diazenyl]phenyl}(ethyl)amino]ethyl hydrogen sulfate, sodium 2-[{4-[(*E*)-{4-chloro-5-[(1,3-dioxo-1,3-dihydro-2*H*-inden-2-ylidene)methyl]-1,3-thiazol-2-yl}diazenyl]phenyl}(ethyl)amino]ethyl sulfate and 2-[{4-[(*E*)-(4-chloro-5-formyl-1,3-thiazol-2-yl)diazenyl] phenyl}(ethyl)amino]ethyl hydrogen sulfate.

**9.** The agent as claimed in one of claims 1 to 8, **characterized in that** the dye of formula (I) and (II) is contained in a total amount of from 0.01 to 10 percent by weight.

**10.** The agent as claimed in one of claims 1 to 9, **characterized in that** it additionally comprises at least one further direct dye which is selected from the group consisting of nitro dyes, azo dyes, anthraquinone dyes, triphenylmethane dyes, basic dyes, acidic dyes and naturally occuring dyes.

**11.** The agent as claimed in claim 10, **characterized in that** the additional direct dye is present in a total amount of from 0.01 to 5 percent by weight.

**12.** The agent as claimed in one of claims 1 to 11, **characterized in that** it has a pH of from 2 to 11.

**13.** The agent as claimed in one of claims 1 to 12, **characterized in that** it is a hair colourant.

European Patent Office

# EUROPEAN SEARCH REPORT

Application Number
EP 06 12 2740

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | DE 101 18 271 A1 (HENKEL KGAA [DE]) 14 March 2002 (2002-03-14) * compound D1 * ----- | 1-13 | INV. A61K8/49 A61Q5/06 A61Q5/10 C09B29/00 |
| A | DE 10 2004 008604 A1 (WELLA AG [DE]) 1 September 2005 (2005-09-01) * table 1 * ----- | 1-13 | |
| A | EP 1 618 865 A (OREAL [FR]) 25 January 2006 (2006-01-25) * page 7, line 13 - line 28 * ----- | 1-13 | |

TECHNICAL FIELDS SEARCHED (IPC)
A61K
A61Q
C09B

The present search report has been drawn up for all claims

| Place of search | Date of completion of the search | Examiner |
|---|---|---|
| Munich | 13 June 2007 | Werner, Stefan |

3

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT ON EUROPEAN PATENT APPLICATION NO. EP 06 12 2740

This annex lists the patent family members relating to the patent documents cited in the above-mentioned European search report.
The members are as contained in the European Patent Office EDP file on
The European Patent Office is in no way liable for these particulars which are merely given for the purpose of information.

13-06-2007

| Patent document cited in search report | Publication date | Patent family member(s) | Publication date |
|---|---|---|---|
| DE 10118271 A1 | 14-03-2002 | NONE | |
| DE 102004008604 A1 | 01-09-2005 | EP 1722754 A1 | 22-11-2006 |
| | | WO 2005079732 A1 | 01-09-2005 |
| EP 1618865 A | 25-01-2006 | FR 2872035 A1 | 30-12-2005 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2005117816 A1
- DE 102004056403 A1
- DE 102004051072 A1
- EP 0450438 A1


### Non-patent literature cited in the description


- **GRIFFITH ; RIEPL.** *Chem. Commun.,* 1998, 1349-1350
- *Dyes and Pigments,* 1995, vol. 27 (1), 45-54
- *Dyes and Pigments,* 1998, vol. 37 (4), 283-289
- **KASALI A. BELLO.** *Dyes and Pigments,* 1995, vol. 27 (1), 45-54
- **MASAKI MATSUI et al.** *Dyes and Pigments,* 1998, vol. 37 (4), 283-289
- **JOHN GRIFFITH et al.** *Chem. Commun.,* 1998, 1349-1350
- *Advanced Organic Chemistry - Reactions, Mechanisms and Structure,* 2001, 368-375
- FIEDLER - Lexikon der Hilfsstoffe. **FIEDLER.** Dictionary of Adjuvants. 2002, vol. 1, 97-102